# EUROPEAN PATENT SPECIFICATION

(11) **EP 2 029 574 B1**
(45) Date of publication and mention of the grant of the patent: **01.12.2010**
(21) Application number: 07748211.5
(22) Date of filing: 07.06.2007
(51) Int. Cl.: C07D 401/12, A61K 31/4439, A61P 1/04

(54) **NOVEL METHOD FOR PREPARATION OF AMMONIUM SALTS OF ESOMEPRAZOLE**
NEUES VERFAHREN ZUR HERSTELLUNG VON AMMONIUMSALZEN VON ESOMEPRAZOL
NOUVEAU PROCÉDÉ POUR PRÉPARER DES SELS D'AMMONIUM D'ÉSOMÉPRAZOLE

(30) Priority: 07.06.2006 US 811698 P
(43) Date of publication of application: 04.03.2009
(73) Proprietor: AstraZeneca AB, 151 85 Södertälje (SE)
(72) Inventor: BERGMAN, Rolf, S-431 83 Mölndal (SE); FREGLER, Christina, S-431 83 Mölndal (SE); LINDBERG, Per, S-431 83 Mölndal (SE)
(86) International application number: PCT/SE2007/000548
(87) International publication number: WO 2007/142580

(56) References cited:
- WO-A-2004/037253
- WO-A1-03/074514
- WO-A1-2005/023796
- WO-A1-2005/023797
- GB-A- 2 137 616

## Description

### Field of the invention

The present invention relates to a process for synthesis of salts of (*S*)-5-methoxy-2-[[(4-methoxy-3,5-dimethyl-2-pyridinyl)-methyl]sulfinyl]-1*H-*benzimidazole (esomeprazole), in a pure and isolated form.

### Background of the invention and prior art

The compound 5-methoxy-2-[[(4-methoxy-3 ,5-dimethyl-2-pyridinyl)metllyl]sulfinyl]-1*H-*benzimidazole, having the generic name omeprazole, and therapeutically acceptable salts thereof, are described in EP 0 005 129.

Omeprazole is a sulfoxide and a chiral compound, wherein the sulphur atom being the stereogenic center. Thus, omeprazole is a racemic mixture of its two single enantiomers, the *R*- and *S*-enantiomer of omeprazole, herein referred to as *R*-omeprazole and *S-*omeprazole, the latter have the generic name esomeprazole. The absolute configuration of the enantiomers of omeprazole has been determined by an X-ray study of an N-alkylated derivate of the *R*-enantiomer.

Omeprazole and esomeprazole are proton pump inhibitors, and are useful as antiulcer agents. In a more general sense, omeprazole and esomeprazole may be used for prevention and treatment of gastric acid related diseases in mammals and especially in man.

Specific alkaline salts of omeprazole are disclosed in EP 0 124 495. Herein, quaternary ammonium salts and guanidine salts of omeprazole are disclosed. Document WO 97/41114 discloses processes for preparing magnesium salt of benzimidazoles, including magnesium salt of omeprazole.

Certain salts of the single enantiomers of omeprazole and their preparation are disclosed in WO 94/27988, for instance, quaternary ammonium salts of esomeprazole are mentioned. The described salts of esomeprazole have improved pharmacokinetic and metabolic properties, which will give an improved therapeutic profile such as a lower degree of interindividual variation. WO 96/02535 and WO 98/54171 disclose preferred processes for preparing esomeprazole and salts thereof.

In the formulation of drug compositions, it is important for the active pharmaceutical ingredient to be in a form in which it can be conveniently handled and processed. This is of importance, not only from the point of view of obtaining a commercially viable manufacturing process, but also from the point of view of subsequent manufacture of pharmaceutical formulations (e.g. oral dosage forms such as tablets) comprising the active pharmaceutical ingredient.

Further, in the manufacture of oral pharmaceutical compositions, it is important that a reliable, reproducible and constant plasma concentration profile of the active pharmaceutical ingredient is provided following administration to a patient.

Chemical stability, solid state stability, and "shelf life" of the active pharmaceutical ingredient are important properties for a pharmaceutical active compound. The active pharmaceutical ingredient, and compositions containing it, should be capable of being effectively stored over appreciable periods of time, without exhibiting a significant change in the physico-chemical characteristics of the active pharmaceutical ingredient, e.g. its chemical composition, density, hygroscopicity and solubility.

### Description of the invention

The present invention refers to a process for preparing a quartenary ammonium salt of esomeprazole of formula I 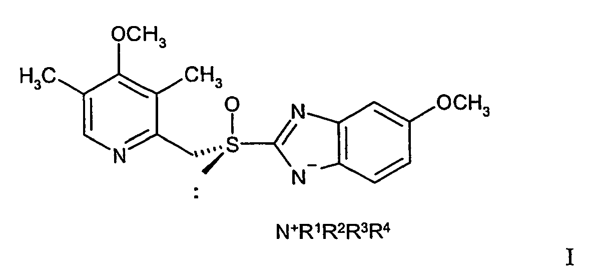 wherein
R₁, R₂, R₃ and R₄ are individually selected from
(A) C₁-C₁₄ alkyl group, which alkyl group is optionally substituted by one or more groups selected from amino, hydroxy, halogen, R₅O-, C₃-C₁₂ cycloalkyl (which cycloalkyl is optionally substituted by one or more groups selected from C₁-C₃ alkyl, hydroxy, C₁-C₃ alkoxy, halogen, oxo, R₂₃ₐOC(O)-, (R_{23b})(R_{23c})NC(O)-, R_{23d}C(O)N(R₂₃ₑ)-,
   R_{23f}C(O)O-, R_{23g}OC(O)-NH-, (R₂₃ₕ)(R₂₃ⱼ)NC(O)O-), aryl or Het¹ (both groups optionally substituted by one to three groups selected from C₁-C₇ alkyl, hydroxy, -CH₂OH, halogen, oxo, nitro, C₁-C₇ alkoxy, R₂₄ₐOC(O)-, (R_{24b}) (R_{24c})NC(O)-, R_{24d}C(O)NCR₂₄ₑ)-, R_{24f}C(O)O-, R_{24g}OC(O)-NH-, (R₂₄ₕ)(R₂₄ⱼ)NC(O)O-, aryl, Het³ or R₂₅C(O)- (which aryl and Het³ are optionally substituted by one or two halogens, C₁-C₄ alkyl, hydroxy C₁-C₄alkyl, C₁-C₄ alkoxy, hydroxy C₁-C₄ alkoxy, nitro); R₆ -O-(CH₂)ₘ-O-, R₇ₐOC(O)-, (R_{7b})(R_{7c})NC(O)-, R_{7d}C(O)N(R₇ₑ)-, R_{7f}C(O)O-, R_{7g}C(O)S-, R₇ₕOC(O)N(R₇ⱼ)-, CR₇ₖ)(R₇ₗ)NC(O)O-, R₇ₘOC(O)O-, R₈-SO₂-NH-, phtalimido, succinimido, R₉C(O)-, R₁₀-(CH₂)ₙ- C(O)- or (R₁₁ₐ)(R_{11b})(R_{11c})C-C(O)O- ;
(B) aryl or Het² (both groups optionally substituted by one to three groups selected from C₁-C₇ alkyl, hydroxy, C₁-C₇ alkoxy, halogen, R₁₂ₐOC(O)-, (R_{12b})(R_{12c})NC(O)-, R_{12d}C(O)N(R₁₂ₑ)-, R_{12f}C(O)O-, R_{12g}OC(O)NR₁₂ₕ-, (R₁₂ⱼ)(R₁₂ₖ)NC(O)O-, aryl, benzoyl or Het⁴), R₁₃C(O)- or (R₁₄ₐ)(R_{14b})N-);
or R₁ and R₂ together may represent a cyclic structure containing 5-14 members, optionally substituted by one or more groups selected from hydroxy, oxo, C₁-C₇ alkyl (which alkyl group is optionally substituted by one or more groups selected from hydroxy, halogen, aryl or Het⁷), R₁₅O-, R₁₆ₐOC(O)-, (R_{16b})(R_{16c})NC(O)-, R_{16d}C(O)N(₁₆ₑ)-, R_{16f}C(O)O-, R_{16g}OC(O)NR₁₆ₕ-, (R₁₆ⱼ)(R₁₆ₖ)NC(O)O-, R₁₇C(O)-, aryl or Het⁵ (which aryl or Het⁵ are optionally substituted by one or more of C₁-C₇ alkyl, hydroxy, oxo, C₁-C₇ alkoxy, halogen, R₂₆ₐOC(O)-, (R_{26b})(R_{26c})NC(O)-, R_{26d}C(O)N(R₂₆ₑ)-, R_{26f}C(O)O-, R_{26g}OC(O)NH-, (R₂₆ₕ)(R₂₆ⱼ)NC(O)O-, phenyl or benzoyl (which phenyl or benzoyl are optionally substituted by one or two halogens or C₁-C₅ alkyl C(O)O-) ), phtalimido, succinimido or (R₁₈ₐ)(R_{18b})(R_{18c})C(O)O-;
or R₁, R₂ and R₃ together may represent a cyclic structure containing 5-16 members, optionally substituted by one or more groups selected from hydroxy, oxo, C₁-C₇ alkyl (which alkyl group is optionally substituted by one or more groups selected from hydroxy, halogen, oxo, aryl or Het⁸), R₁₉O-, R₂₀C(O)-, aryl or Het⁶ (which aryl or Het⁶ are optionally substituted by one to three groups selected from C₁-C₇ alkyl, hydroxy, C₁-C₇ alkoxy, halogen, oxo, R₂₇ₐOC(O)-, (R_{27b})(R_{27c})NC(O)-, R_{27d}C(O)N(R₂₇ₑ)-, R_{27f}C(O)O-, R_{27g}OC(O)-NH-, (R₂₇ₕ)(R₂₇ⱼ)NC(O)O-, phenyl or benzoyl), R₂₁ₐOC(O)-, (R_{21b})(R_{21c})NC(O)-, R_{21d}C(O)N(R₂₁ₑ)-, R_{21f}C(O)O-, R_{21g}OC(O)-NR₂₁ₕ-, (R₂₁ⱼ)(R₂₁ₖ)NC(O)O-, phtalimido, succinimido or (R₂₂ₐ)(R_{22b})(R_{22c}) C-C(O)O-;
wherein
R₅ is selected from C₁-C₆ alkyl, aryl, Het⁹ (which groups are optionally substituted by one or more groups selected from hydroxy, halogen, C₁-C₆ alkoxy);
R₆ is selected from aryl or Het¹⁰ (both groups optionally substituted by one or more groups selected from C₁-C₈ alkyl, hydroxy, C₁-C₇ alkoxy, halogen, R₂₈ₐOC(O)-, (R_{28b})(R_{28c})NC(O)-, R_{28d}C(O)N(R₂₈ₑ)-, R_{28f}(O)O- R_{28g}OC(O)-NH-, (R₂₈ₕ)(R₂₈ⱼ)NC(O)O-, aryl, benzoyl or Het¹¹);
R₇ₐ to R₇ₘ are independently selected, at each occurrence, from hydrogen, C₁-C₇ alkyl, aryl or Het¹² (which C₁-C₇ alkyl, aryl and Het¹² are optionally substituted by one or more groups selected from C₁-C₆ alkyl, hydroxy, C₁-C₃ alkoxy, halogen, R₂₉ₐOC(O)-, (R_{29b})(R_{29c})NC(O)-, R_{29d}C(O)N(R₂₉ₑ)-, R_{29f}C(O)O-, R_{29g}OC(O)-NH-, (R_{29b})(R₂₉ⱼ)NC(O)O-, aryl, benzoyl or Het¹³)_{;}
R₈ is selected from C₁-C₆ alkyl, aryl or Het¹⁴ (which groups are optionally substituted by one or more groups selected from C₁-C₆ alkyl);
R₉ is selected from linear or branched C₁ - C₁₂ alkyl (optionally substituted by R₃₀OC(O)- ), C₃-C₁₂ cycloalkyl (which cycloalk-yl group is optionally further substituted by one or more groups selected from C₁-C₃ alkyl, hydroxy, C₁-C₃ alkoxy, halogen, R₃₁ₐOC(O)-, (R_{31b})(R_{31c})NC(O)-, R_{31d}C(O)NR₃₁ₑ-, R_{31f}C(O)O-, R_{31g}C(O)N(R₃₁ₕ)-, (R₃₁ⱼ)(R₃₁ₖ)NC(O)O-), aryl, benzoyl or Het¹⁵), aryl or het¹⁶ (which aryl and Het¹⁶ are optionally substituted by one to three of the groups selected from C₁-C₆ alkyl, hydroxy, C₁-C₃ alkoxy, ethylenedioxy, halogen, R₃₂ₐOC(O)-, (R_{32b})(R_{32c})NC(O)-, R_{32d}C(O)NR₃₂ₑ-, R_{32f}(O)O-, R_{32g}OC(O)NH-, (R₃₂ₕ)(R₃₂ⱼ)NC(O)O-), aryl, benzoyl or Het¹⁷);
R₁₀ is selected from aryl and Het¹⁸ (which groups are optionally substituted by one to three groups selected from C₁-C₃ alkyl, hydroxy, C₁-C₃ alkoxy, halogen, -COOH, ethylenedioxy);
R₁₁ₐ is selected from hydroxy or-CH₂OH;
R_{11b} is phenyl (optionally substituted by one to three groups selected from C₁-C₃ alkyl, hydroxy, C₁-C₃ alkoxy, halogen, R₃₃ₐOC(O)-, (R_{33b})(R_{33c})NC(O)-, R_{33d}C(O)N(R₃₃ₑ)-, R_{33f}C(O)O-, R_{33g}OC(O)-NH-, (R₃₃ₕ)(R₃₃ⱼ)NC(O)O-;
R_{11c} is selected from hydrogen, C₅-C₆ cycloalkyl or phenyl (which groups are optionally substituted by one to three groups selected from C₁-C₃alkyl, hydroxy, C₁-C₃alkoxy, halogen, R₃₄ₐOC(O)-, (R_{34b})(R_{34c})NC(O)-, R_{34d}C(O)N(R₃₄ₑ)-, R_{34f}C(O)O-, R_{34g}OC(O)NH-, (R₃₄ₕ)(R₃₄ⱼ)NC(O)O-);
R₁₂ₐ to R₁₂ₖ are independently selected, at each occurrence, from hydrogen, C₁-C₇alkyl, aryl, Het¹⁹ (which groups are optionally substituted by one or more groups selected from C₁-C₆alkyl, hydroxy, C₁-C₃alkoxy, halogen, R₃₅ₐOC(O)-, (R_{35b})(R_{35c})NC(O)-, R_{35d}C(O)N(R₃₅ₑ)-, R_{35f}C(O)O-, R_{35g}OC(O)-NH-, (R₃₅ₕ)(R₃₅ⱼ)NC(O)O-, aryl, benzoyl or Het²⁰);
R₁₃ is selected from hydrogen or C₁-C₆ alkyl;
R₁₄ₐ to R_{14b} are independently selected, at each occurrence, from hydrogen or C₁-C₆ alkyl,;
R₁₅ is selected from C₁-C₆ alkyl, aryl or Het²¹ (which groups are optionally substituted by one or more groups selected from hydroxy, halogen or C₁-C₆ alkoxy);
R₁₆ₐ to R₁₆ₖ are independently selected from, at each occurrence, hydrogen, C₁-C₇ alkyl, aryl or Het²² (which groups are optionally substituted by one or more groups selected from C₁-C₆ alkyl, hydroxy, C₁-C₃ alkoxy, halogen, R₃₆ₐOC(O)-, (R_{36b})(R_{36c})NC(O)-,
R_{36d}C(O)N(R₃₆ₑ)-, R_{36f}C(O)O-, R_{36g}OC(O)-NH-, (R₃₆ₕ)(R₃₆ⱼ)NC(O)O-, aryl, benzoyl or Het²³);
R¹⁷ is selected from hydrogen or C₁-C₆ alkyl;
R₁₈ₐ is selected from hydroxy or-CH₂OH;
R_{18b} is phenyl (optionally substituted by one to three groups selected from C₁-C₃ alkyl, hydroxy, C₁-C₃ alkoxy, halogen, R₃₇ₐOC(O)-, (R_{37b})(R_{37c})NC(O)-, R_{37d}C(O)N(R₃₇ₑ)-, R_{37f}C(O)O-, R_{37g}OC(O)-NH-, (R₃₇ₕ)(R₃₇ⱼ)NC(O)O-);
R_{18c} is selected from hydrogen, C₅-C₆ cycloalkyl or phenyl (which groups are optionally substituted by one to three groups selected from C₁-C₃ alkyl, hydroxy, C₁-C₃ alkoxy, halogen, R₃₈ₐOC(O)-, (R_{38b})(R_{38c})NC(O)-, R_{38d}C(O)N(R₃₈ₑ)-, R_{38f}C(O)O-, R_{38g}OC(O)-NH-, (R₃₈ₕ)(R₃₈ⱼ)NC(O)O-;
R₁₉ is selected from C₁-C₆ alkyl, aryl or Het²⁴ (which groups are optionally substituted by one or more groups selected from hydroxy, halogen, C₁-C₆ alkoxy);
R₂₀ is selected from hydrogen and C₁-C₆ alkyl;
R₂₁ₐ to R₂₁ₖ are independently selected, at each occurence, from hydrogen, C₁-C₇ alkyl, aryl or Het²⁵ (which groups are optionally substituted by one or more groups selected from C₁-C₆ alkyl, hydroxy, C₁-C₃ alkoxy, halogen, R₃₉ₐOC(O)-, (R_{39b})(R_{39c})NC(O)-, R_{39d}C(O)N(R₃₉ₑ)-, R_{39f}C(O)O-, R_{39g}OC(O)-NH-, (R₃₉ₕ)(R₃₉ⱼ)NC(O)O-, aryl, benzoyl or Het²⁶);
R₂₂ₐ is selected from hydroxy or -CH₂OH;
R_{22b} is phenyl (optionally substituted by one to three groups selected from C₁-C₃ alkyl, hydroxy, C₁-C₃ alkoxy, halogen, R₄₀ₐOC(O)-, (R_{40b})(R₄₀c)NC(O)-, R_{40d}C(O)N(R₄₀ₑ)-, R_{40f}C(O)O-, R_{40g}OC(O)NH-, (R₄₀ₕ)(R₄₀ⱼ)NC(O)O-);
R_{22c} is selected from hydrogen, C₅-C₆ cycloalkyl or phenyl (which optionally is substituted by one to three groups selected from C₁-C₃ alkyl, hydroxy, C₁-C₃ alkoxy, halogen, R₄₁ₐOC(O)-, (R_{41b})(R_{41c})NC(O)-, R_{41d}C(O)N(R₄₁ₑ)-, R_{41f}C(O)O-, R_{41g}OC(O)NH-, (R₄₁ₕ)(R₄₁ⱼ)NC(O)O-);
R₂₃ₐ to R₂₃ⱼ are independently selected, at each occurrence, from hydrogen or C₁-C₆alkyl; R₂₄ₐ to R₂₄ⱼ are independently selected, at each occurrence, from hydrogen or C₁-C₆alkyl; R₂₅ is selected from C₁-C₄alkyl, aryl or Het²⁷ (which aryl and Het²⁷ are optionally substituted by one or two halogens, C₁-C₄ alkyl, hydroxy C₁-C₄alkyl, C₁-C₄ alkoxy, hydroxy C₁-C₄ alkoxy, nitro);
R₂₆ₐ to R₂₆ⱼ are independently selected, at each occurrence, from hydrogen or C₁-C₆alkyl;
R₂₇ₐ to R₂₇ⱼ are independently selected, at each occurrence, from hydrogen or C₁-C₆alkyl;
R₂₈ₐ to R₂₈ⱼ are independently selected, at each occurrence, from hydrogen or C₁-C₆alkyl;
R₂₉ₐ to R₂₉ⱼ are independently selected, at each occurrence, from hydrogen or C₁-C₆alkyl;
R₃₀ is selected from hydrogen or C₁-C₆ alkyl;
R₃₁ₐ to R₃₁ₖ are independently selected, at each occurrence, from hydrogen or C₁-C₆alkyl;
R₃₂ₐ to R_{32c} are independently selected, at each occurrence, from hydrogen or C₁-C₆akyl;
R₃₃ₐ to R₃₃ⱼ are independently selected, at each occurrence, from hydrogen or C₁-C₆alkyl;
R₃₄ₐ to R₃₄ⱼ are independently selected, at each occurrence, from hydrogen or C₁-C₆alkyl;
R₃₅ₐ to R₃₅ⱼ are independently selected, at each occurrence, from hydrogen or C₁-C₆alkyl;
R₃₆ₐ to R₃₆ⱼ are independently selected, at each occurrence, from hydrogen or C₁-C₆alkyl;
R₃₇ₐ to R₃₇ⱼ are independently selected, at each occurrence, from hydrogen or C₁-C₆alkyl;
R₃₈ₐ to R₃₈ⱼ are independently selected, at each occurrence, from hydrogen or C₁-C₆alkyl;
R₃₉ₐ to R₃₉ⱼ are independently selected, at each occurrence, from hydrogen or C₁-C₆alkyl;
R₄₀ₐ to R₄₀ⱼ are independently selected, at each occurrence, from hydrogen or C₁-C₆alkyl;
R₄₁ₐ to R₄₁ⱼ are independently selected, at each occurrence, from hydrogen or C₁-C₆alkyl;
m is an integer selected from 1 to 5;
n is an integer selected from 1 to 3.

The process for preparing the quaternary ammoniumsalts esomeprazole of formula I comprises the following steps:
(i): mixing esomeprazole and N⁺ (R₁)(R₂)(R₃)(R₄) X⁻;
   wherein R₁, R₂, R₃ and R₄ are as defined above; X⁻ is selected from Cl⁻, Br , I⁻, carboxylates, sulphonates, HSO₄⁻ and OH⁻;
   in an aqueous solvent system substantially saturated with potassium carbonate;
(ii): adding a water immiscible chlorinated hydrocarbon solvent;
(iii): isolating the organic phase;
(iv): recovering of the compound of formula I.

In one embodiment of the invention the reaction of the esomeprazole and the N⁺(R₁)(R₂)(R₃)(R₄) X⁻ as defined above is performed in an aqueous solvent solvent substantially saturated with potassium carbonate (K₂CO₃).

By "substantially saturated" it is meant a solution comprising equal or more than 40 % by weight potassium carbonate in an aqueous solvent, for example more than 45, 50 or 55 % by weight.

In one embodiment of the invention, the aqueous solvent system in step (i) is saturated with potassium carbonate, i.e. comprises about 56 % by weight potassium carbonate.

In one embodiment of the invention the esomeprazole and the quartenary ammoniumsalt of formula N⁺(R₁)(R₂)(R₃)(R₄) X⁻ are in step (i) added in equimolar amounts.

The aqueous solvent system may be selected from water or water soluble solvents, such as alcohols, ethers, amides, nitriles soluble in water; or mixtures thereof. Examples of water soluble solvents are methanol, ethanol, dioxane, tetrahydrofuran, acetonitril and DMF.

In one embodiment the aqueous solvent system is water.

The water immiscible solvent forming the organic phase are selected from solvents such as chlorinated solvents suitable for phase transfer. The solvent must also be stable in the presence of base, i.e. for the present invention the solvent should not degrade more than to some extent in the presence of the potassium carbonate. Examples of chlorinated solvents are dichloromethane, trichloromethane and 1,2-dichloroethane.

Unless otherwise specified, alkyl groups and alkoxy groups as defined herein may be linear or, when there is a sufficient number (i.e. a minimum of three) of carbon atoms be branched, and/or cyclic.

As used herein, the term "C₁-C₁₄ alkyl group" is an alkyl group having 1 to 14 carbon atoms. Examples of said group includes, but is not limited to, methyl, ethyl, propyl, butyl, pentyl, hexyl, heptyl, octyl, nonyl and dekanyl and when the alkyl is branched, iso-propyl, iso-butyl, sec-butyl, tert-butyl, sec-pentyl, iso-pentyl and neo-pentyl.

The term "C₃-C₁₂ cycloalkyl" is a cyclic alkyl group having 3 to 12 carbon atoms. The cyclic group may be a mono, di or polycyclic-group, and it may optionally be substituted with 1, 2, or 3 methyl groups. Examples of said cyclic alkyl group includes, but is not limited to, cyclopropyl, cyclobutyl, cyclopentyl, cyclohexyl and adamantyl.

Unless otherwise specified, the alkyl and alkoxy groups may also be substituted by one or more fluoro atoms. Examples of said substituted alkyl or alkoxy groups are trifluoromethyl, trifluoromethoxy and trifluoroethyl.

Alkylene groups as defined herein are divalent and may be linear or, when there is a sufficient number (i.e. a minimum of three) of carbon atoms, be branched. Unless otherwise specified, alkylene groups may also be substituted by one or more halogen atoms, and especially fluoro atoms.

The term "aryl", when used herein, includes C₆-C₁₀ aryl groups such as phenyl, naphtyl, and the like. Unless otherwise specified, the aryl group may be substituted by one or more substituents including -OH, cyano, nitro, C₁-C₇ alkoxy, C₁-C₇ alkyl, halogen for example fluoro. Examples are phenyl substituted by one, two or three halogens such as fluoro.

Unless otherwise specified the term "benzoyl" also includes benzoyl groups which may be substituted by one or more halogen, for example fluoro.

Het groups (Het¹ to Het²⁷) that may be mentioned include those ring systems having a total number of atoms in the ring system or between five and twelwe atoms and containing 1 to 5 heteroatoms (selected from N, O and S). Het groups may be fully saturated, wholly aromatic, partly aromatic and/or bi- or polycyclic in character. Heterocyclic groups that may be mentioned include benzodioxanyl, benzodioxepanyl, benzodioxolyl, benzofuranyl, benzimidazolyl, benzomorpholinyl, benzoxazinonyl, benzothiophenyl, chromanyl, cinnolinyl, dioxanyl, furanyl, imidazolyl, imidazo[1,2-a]pyridinyl, indolyl, isoquinolinyl, isoxazolyl, morpholinyl, oxazolyl, phthalazinyl, piperazinyl, piperidinyl, purinyl, pyranyl, pyrazinyl, pyrazolyl, pyridinyl, pyrimindinyl, pyrrolidinonyl, pyrrolidinyl, pyrrolinyl, pyrrolyl, quinazolinyl, quinolinyl, tetrahydropyranyl, tetrahydrofuranyl, thiazolyl, thienyl, thiochromanyl, triazolyl, xanthanyl and the like. Substituents on Het groups may, where appropriate, be located on any atom in the ring system including a heteroatom. The point of attachment of Het groups may be *via* any atom in the ring system including (where appropriate) a heteroatom, or an atom on any fused carbocyclic ring that may be present as part of the ring system. Het groups may also be in the N- or S-oxidised form. Unless otherwise specified, the Het group may be substituted by one or more substituents including -OH, cyano, nitro, C₁-C₇ alkoxy, C₁-C₇ alkyl, halogen for example fluoro.

The term "halogen", when used herein, includes fluoro, chloro, bromo and iodo.

The phrase "R₁ and R₂ together may represent a cyclic structure containing 5-14 members" means that a mono-, bi-, tri- or polycyclic structure containing 5-14 atoms, of which optionally 1 to 5 are heteroatoms selected from N, O and S is formed. The cyclic structure may contain one or more double bond, and which cyclic structure may have one or more condensed aryl or Het. The cyclic structure may be further substituted. Examples of compounds included are pyrrolidine, piperidine, azepane, piperidone, piperazine, morpholine, tetrahydropyridine, imidazole, imidazoline, isoindoline, tetrahydroisoquinoline, carbazole, 6,7-dihydro-5*H-*dibenzo[c,e]azepine, 8-aza-bicyclo[3,2,1]octane, desmethyltropine, 3-oxa-9-aza-tricyclo[3.3.1.0*2,4*]nonane and desmethylscopine.

The phrase "R₁, R₂ and R₃ together may represent a cyclic structure containing 5-16 members" means that R₁, R₂ and R₃ together form a tri-, tetra- or polycyclic structure containing 5 to 16 atoms, of which optionally 1 to 5 are heteroatoms selected from N, O and S. The cyclic structure may contain one or more double bond, and which cyclic structure may have a condensed aryl or Het and which cyclic structure may optionally be further substituted by one or more groups. Examples of structures included are hexamethylenetetramine and quinuclidine.

The N⁺(R₁)(R₂)(R₃)(R₄) X added in step (i) is defined to be salts of Cl⁻, Br⁻, I⁻, carboxylates, sulphonates, HSO₄⁻ and OH⁻. Examples of carboxylates are aliphatic carboxylic acids, for example C₁-C₆ alkyl carboxylic acid, such as acetic acid and propionic acid; of sulphonates are alkylsulphonates, for example C₁-C₆ alkyl sulphonates such as methane-, ethane- or propanesulphonic acid.

In one embodiment of the invention, the compound of formula I provided by the process above is
tetra-n-butyl ammoniumsalt of esomeprazole;
cholin salt of esomeprazole;
benzyltrimethylammonium salt of esomeprazole;
(1*S*)-N,N,N,trimethyl-1-phenylethylammonium salt of esomeprazole;
(1*R*,2*S*)-N,N-dimethylephedrinium salt of esomeprazole;
(1*S*, 2*R*)-N,N-dimethylephedrinium salt of esomeprazole;
(1*R*, 2*S*)-N-benzyl-N-methylephedrinium salt of esomeprazole;
(1*S*, 2*R*)-N-benzyl-N-methylephedrinium salt of esomeprazole or
cis-2,6-dimethyl-N,N-dimethylpiperidinium salt of esomeprazole.

Due to tautomerism the chemical name (*S*)-5-methoxy-2-[[(4-methoxy-3,5-dimethyl-2-pyridinyl)methyl]sulfinyl]-1*H-*benzimidazole ammonium salt does not necessarily mean that the methoxy group of the two benzimidazole moieties is in the 5-position but may as well be in the 6-position, or there may be mixtures of the two.

The compounds of formula I may be prepared in the form of solvates, hydrates, and anhydrates.

The esomeprazole mixed in step (i) of the process of the invention is the neutral form esomeprazole, or the sodium salt or potassium salt of esomeprazole.

The process of the present invention is advantageous because of its simplicity. The process of the present of invention is defined by increased ease of handling including improved phase separation in step (iii) and an inherent drying off effect. During a phase transfer, most often a small amount of water remains in the organic phase. However, the presence of potassium carbonate in the present process reduces or even eliminates the remaining parts of aqueous solvent system in the organic phase, and thus also the need for a following drying step. It further gives products of high purity.

The compounds of formula I are effective as gastric acid secretion inhibitors, and are thus useful as antiulcer agents. In a more general sense, they can be used for prevention and treatment of gastric-acid related conditions in mammals and especially in man, including e.g. reflux esophagitis, gastritis, duodenitis, gastric ulcer and duodenal ulcer. Furthermore, they may be used for treatment of other gastrointestinal disorders where gastric acid inhibitory effect is desirable e.g. in patients on NSAID therapy, in patients with Non Ulcer Dyspepsia, in patients with symptomatic gastro-esophageal reflux disease, and in patients with gastrinomas. They may also be used in patients in intensive care situations, in patients with acute upper gastrointestinal bleeding, pre- and postoperatively to prevent aspiration of gastric acid, to prevent and treat stress ulceration and asthma, and for improvement of sleep. Further, the compounds of the invention may be useful in the treatment of psoriasis as well as in the treatment of Helicobacter infections and related diseases. The compounds of formula I may also be used for treatment of inflammatory conditions in mammals, including man.

Any suitable route of administration may be employed for providing the patient with an effective dosage of the quartenary ammoniumsalt of esomeprazole. For example, peroral or parenteral formulations, including i.v., and the like may be employed. Dosage forms include capsules, tablets, dispersions, suspensions, solutions and the like.

In the practice of the invention, the most suitable route of administration as well as the magnitude of the therapeutic dose will depend on the nature and severity of the disease to be treated. The dose, and dose frequency, may also vary according to the age, body weight and response of the individual patient. Special requirements may be needed for patients having Zollinger-Ellison syndrome, such as a need for higher doses than the average patient. Children and patients with liver diseases generally will benefit from doses that are somewhat lower than average. Thus, in some conditions it may be necessary to use doses outside the ranges stated below, for example long-term treatments may request lower dosage. Such higher and lower doses are within the scope of the present invention. Such daily doses may vary between 5 mg to 300 mg.

In general, a suitable oral dosage form of the compound of the invention may cover a dose range from 5 mg to 300 mg total daily dose, administered in one single dose or equally divided doses. A preferred dosage range is from 10 mg to 80 mg.

### Examples

*The examples below will further illustrate the preparation of the compound of the invention. These examples are not intended to limit the scope if the invention as defined hereinabove or as claimed below.*

The quaternary ammoniumsalt of formula N⁺(R₁)(R₂)(R₃)(R₄)Cl⁻ as defined above may be commercially available or otherwise synthesized according to the methods described below in Example A to Example F.

### Example A (Reference)

### Preparation of (1S)-N,N,N- trimethyl-1-phenethylammonium chloride

(1S)-N,N-dimethyl-1-phenetylamine (0.61g, (4 mmol)) was dissolved in acetone (20ml) and methyl iodide (2g (14 mmol)) was added. The flask was sealed and the mixture was left over night at ambient temperature before it was diluted with diethyl ether (50 ml). The crystalline salt was filtered off and washed with diethyl ether. The quaternary ammonium iodide was dissolved in water (deionised) and the solution was filtered through an anion exchanger (50ml Amberlite IRA-400; 20-50 mesh; Cl⁻-form) and eluted with deionized water. The eluate was concentrated to ca 20 ml at reduced pressure and freeze drying gave 600 mg (3 mmol) of crystalline (1S)-N,N,N- trimethyl-1-phenetylammonium chloride.

¹H-NMR (400 MHz; CDCl₃): δ 7.59 (m, 1H), 7.43 (m, 3H), 5.33 (q, 2H), 3.34 (s, 9H), 1.81 (δ, 3H).

### Example B (Reference)

### Preparation of (1R, 2S)-N,N-dimethylephedrinium chloride

(1*R*,2*S*)-N-methylephedrin (0.72g, (4 mmol)) was dissolved in acetone (20ml) and methyl iodide (2g (14 mmol)) was added. The flask was sealed and the mixture was left over night at ambient temperature before it was diluted with diethyl ether (50 ml). The crystalline salt was filtered off and washed with diethyl ether. The quarternary ammonium iodide was dissolved in water (deionised) and the solution was filtered through an anion exchanger (50ml Amberlite IRA-400; 20-50 mesh; Cl⁻-form) and eluted with deionized water. The eluate was concentrated to ca 20 ml at reduced pressure and freeze drying gave 685 mg (3 mmol) of crystalline (1R,2S)-N,N- dimethylephedrinium chloride.

¹H-NMR (400 MHz; CDCl₃): δ 7.27 (m, 2H), 7.22 (m, 2H), 5.42 (s, 1H), 3.46 (m, 1H), 3.19 (m, 1H), 3.16 (s, 9H), 1.08 (m, 3H).

### Example C (Reference)

### Preparation of (1S, 2R)-N,N-dimethylephedrinium chloride

(1*S*, 2*R*)-N-methylephedrin (0.72g, (4 mmol)) was dissolved in acetone (20ml) and methyl iodide (2g (14 mmol)) was added. The flask was sealed and the mixture was left over night at ambient temperature before it was diluted with diethyl ether (50 ml). The crystalline salt was filtered off and washed with diethyl ether. The quarternary ammonium iodide was dissolved in water (deionised) and the solution was filtered through an anion exchanger (50ml Amberlite IRA-400; 20-50 mesh; Cl⁻-form) and eluted with deionized water. The eluate was concentrated to ca 20 ml at reduced pressure and freeze drying gave 850 mg (3.7 mmol) of crystalline (1S,2R)-N,N- dimethylephedrinium chloride.

¹H-NMR (400 MHz; CDCl₃): δ 7.27 (m, 2H), 7.22 (m, 2H), 5.42 (s, 1H), 3.46 (m, 1H), 3.19 (m, 1H), 3.16 (s, 9H), 1.08 (m, 3H).

### Example D (Reference)

### Preparation of (1R, 2S)-N-benzyl-N-methylephedrinium bromide

(1*R*,2*S*)-N-methylephedrin (0.5 g, (2.79 mmol)) was dissolved in dimethoxyethane (5ml) and benzyl bromide (0.6 g (3.5 mmol)) was added. The flask was sealed and the mixture was left over night at ambient temperature before it was diluted with diethyl ether (10 ml). The crystalline salt was filtered off and washed with diethyl ether. Air drying at room temperature gave 0.74 g (2.11 mmol) of the title compound.

¹H-NMR (400 MHz; CDCl₃): δ 7.60 (d, 2H), 7.40 (bm, 5H), 7.19 (m, 2H), 7.12 (m, 1H), 5.95 (d,1H), 5.37 (d, 1H), 5.20 (d, 1H), 4.91 (d, 1H), 3.98 (q, 1H), 3.30 (s, 3H), 3.19 (s, 3H), 1.24 (d, 3H).

### Example E (Reference)

### Preparation of (1S, 2R)-N-benzyl-N-methylephedrinium bromide

(1*S*,2*R*)-N-methylephedrin (0.5 g, (2.79 mmol)) was dissolved in dimethoxyethane (5ml) and benzyl bromide (0.6 g (3.5 mmol)) was added. The flask was sealed and the mixture was left over night at ambient temperature before it was diluted with diethyl ether (10 ml). The crystalline salt was filtered off and washed with diethyl ether. Air drying at room temperature gave 0.75 g (2.14 mmol) of the title compound.

¹H-NMR (400 MHz; CDCl₃): δ 7.60 (d, 2H), 7.40 (bm, 5H), 7.19 (m, 2H), 7.12 (m, 1H), 5.95 (d, 1H), 5.37 (d, 1H), 5.20 (d, 1H), 4.91 (d, 1H), 3.98 (q, 1H), 3.30 (s, 3H), 3.19 (s, 3H), 1.24 (d, 3H).

### Example F (Reference)

### Preparation of cis-2,6-dimethyl-N,N-dimethylpiperidinium iodide

Methyl iodide (2 g (14 mmol)) was added to a mixture of cis-2,6-dimethylpiperidine (0.46 g (4 mmol)), potassium carbonate (anhydrous) (1 g (7.3 mmol)) and water (1 ml) in dichloromethane (8 ml). The mixture was shaken carefully for 10 min whereupon the phases were separated. The organic phase was concentrated to drieness at reduced pressure and the crystalline residue was treated with acetone. Filtration and air drying gave 0.92 g (3.42 mmol) of the title compound.

¹H-NMR (400 MHz; CDCl₃): δ 4.20 (m, 2H), 3.35 (s, 3H), 2.86 (s, 3H), 1.86 (m, 6H), 1.47 (d, 6H).

### Example 1

### Preparation of tetra-n-butylammoniumsalt of esomeprazole

Esomeprazole sodium salt (0.37 g (1 mmol)) was added to a mixture of tetra-n-butylammonium chloride (0.28 g (1 mmol)), potassium carbonate (anhydrous) (1 g (7.3 mmol) and water (1 ml). Dichloromethane (8ml) was added and the mixture was shaken by hand (1 min). After separation, the organic phase was dried over Na₂SO₄ and filtered. The filtrate was concentrated to drieness at reduced pressure. 0.58 g (0.98 mmol) of tetra-n-butylammonium salt of esomeprazole (oil) was obtained.

¹H-NMR (400 MHz; CDCl₃): δ 8.21 (s, 1H), 7.54 (d, 1H), 7.18 (m, 1H), 6.74 (dm, 1H), 4.94 (d, 1H), 4.65 (D, 1H), 3.82 (s, 3H), 3.63 (s, 3H), 2.97 (bm, 8H), 2.19 (s, 3H), 2.18 (s, 3H), 1.29 (bm, 16H), 0.93 (bt,12H).

### Example 2

### Preparation of cholin salt of esomeprazole

Esomeprazole sodium salt (0.37 g (1 mmol)) was added to a mixture of cholin chloride (0.14 g (1 mmol)), potassium carbonate (anhydrous) (1 g (7.3 mmol) and water (1 ml). Dichloromethane (8 ml) was added and the mixture was shaken (1 min). After separation, the organic phase was dried over Na₂SO₄ and filtered. The filtrate was concentrated to drieness at reduced pressure. 0.44 g (0.98 mmol) cholin salt of esomeprazole (amorphous foam) was obtained.

¹H-NMR (400 MHz; CDCl₃): δ 8.14 (s, 1H), 7.50 (d, 1H), 7.10 (m, 1H), 6.79 (dm, 1H), 4.83 (d, 1H), 4.57 (d, 1H), 3.84 (m, 2H), 3.82 (s, 3H), 3.67 (s, 3H), 3.27 (m, 1H), 3.10 (m, 1H), 2.93 (s, 9H), 2.20 (s, 3H), 2.19 (s, 3H).

### Example 3

### Preparation of benzyltrimethylammonium salt of esomeprazole

Esomeprazole sodium salt (0.37 g (1 mmol)) was added to a mixture of benzyl trimethyl ammonium chloride (0.19 g (1 mmol)), potassium carbonate (anhydrous) (1 g (7.3 mmol) and water (1 ml). Dichloromethane (8ml) was added and the mixture was shaken (1 min). After separation, the organic phase was dried over Na₂SO₄ and filtered. The filtrate was concentrated to drieness at reduced pressure. 0.48 g (0.97 mmol) of benzyltrimethylammonium salt of esomeprazole (oil) was obtained.

¹H-NMR (400 MHz; CDCl₃): δ 8.12 (s, 1H), 7.52 (d, 1H), 7.38 (bm, 3H), 7.21 (bd, 2H), 7.14 (m, 1H), 6.74 (dm, 1H), 4.91 (d, 1H), 4.62 (d, 1H), 4.13 (s, 2H), 3.76 (s, 3H), 3.65 (s, 3H), 2.74 (s, 9H), 2.20 (s, 3H), 2.15 (s, 3H).

### Example 4

### Preparation of (1S)-N, N, N, trimethyl-1-phenylethylammonium salt of esomeprazole

Esomeprazole sodium salt (0.37 g (1 mmol)) was added to a mixture of (1S)-N, N, N, trimethyl-1-phenylethylammonium chloride (0.2 g (1 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.50 g (0.98 mmol) of (1S)-N, N, N, trimethyl-1-phenylethylammonium salt of esomeprazole (oil) was obtained.

¹H-NMR (400 MHz; CDCl₃): δ 8.15 (s, 1H), 7.54 (d, 1H), 7.35 (bm, 5H), 7.15 (m, 1H), 6.77 (dm, 1H), 4.92 (d, 1H), 4.66 (d, 1H), 3.78 (s, 3H), 4.57 (q, 1H) 3.65 (s, 3H), 2.84 (s, 9H), 2.20 (s, 3H), 2.17 (s, 3H), 1.54 (d, 3H).

### Example 5

### Preparation of (1R, 2S)-NN-dimethylephedrinium salt of esomeprazole

Esomeprazole sodium salt (0.37 g (1 mmol)) was added to a mixture of (1*R*, 2*S*)-N,N-dimethylephedrinium chloride (0.23 g (1 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.505 g (0.98 mmol) of (1*R*, 2*S*)-N,N-dimethylephedrinium salt of esomeprazole (amorphous foam) was obtained.

¹H-NMR (400 MHz; CDCl₃): δ 8.06 (s, 1H), 7.38 (bm, 3H), 7.26 (bm, 2H), 7.19 (m, 1H), 6.88 (m, 1H), 6.68 (dm, 1H), 5.83 (s, 1H), 4.65 (d, 1H), 4.42 (d, 1H), 3.65 (s, 3H), 3.56 (s, 3H), 3.10 (q, 1H), 2.86 (s, 9H), 2.14 (s, 3H), 1.93 (s, 3H), 1.10 (d, 3H).

### Example 6

### Preparation of (1S, 2R)-N,N-dimethylephedrinium salt of esomeprazole

Esomeprazole sodium salt (0.37 g (1 mmol)) was added to a mixture of (1*S*, 2*R*)-N,N-dimethylephedrinium chloride (0.23 g (1 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.53 g (0.98 mmol) of (1*S*, 2*R*)-N,N-dimethylephedrinium salt of esomeprazole (amorphous foam) was obtained.

¹H-NMR (400 MHz; CDCl₃): δ 8.09 (s, 1H), 7.35 (bm, 3H), 7.28 (bm, 2H), 7.22 (m, 1H), 6.79 (m, 1H), 6.66 (dm, 1H), 5.60 (s, 1H), 4.76 (d, 1H), 4.52 (d, 1H), 3.62 (s, 3H), 3.57 (s, 3H), 3.13 (q, 1H), 2.96 (s, 9H), 2.17 (s, 3H), 2.09 (s, 3H), 1.12 (d, 3H).

### Example 7

### Preparation of (1R, 2S)-N-benzyl-N-methylephedrinium salt of esomeprazole

Esomeprazole sodium salt (0.185 g (0.5 mmol)) was added to a mixture of (1*R*, 2*S*)-N-benzyl-N-methylephedrinium bromide (0.175 g (0.5 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.265 g (0.43 mmol) of (1*R*, 2*S*)-N-benzyl-N-methylephedrinium salt of esomeprazole (amorphous foam) was obtained.

¹H-NMR (400 MHz; CDCl₃): δ 8.00 (s, 1H), 7.49 (d, 1H), 7.44 (d, 2H), 7.39 (t, 1H), 7.27 (bm, 3H), 7.13 (d, 2H), 6.99 (d, 1H), 6.70 (dd, 1H), 6.2 (s, 1H), 4.64 (d, 1H), 4.52 (s, 2H), 4.49 (d, 1H), 3.71 (s, 3H), 3.57 (s, 3H), 3.49 (q, 1H), 2.92 (s, 3H), 2.92 (s, 3H), 2.12 (s, 3H), 1.97 (s, 3H), 1.34 (d, 3H).

### Example 8

### Preparation of (1S, 2R)-N-benzyl-N-methylephedrinium salt of esomeprazole

Esomeprazole sodium salt (0.185 g (0.5 mmol)) was added to a mixture of (1*S*, 2*R*)-N-benzyl-N-methylephedrinium bromide (0.175 g (0.5 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.260 g (0.42 mmol) of (1*S*, 2*R*)-N-benzyl-N-methylephedrinium salt of esomeprazole (amorphous foam) was obtained.

¹H-NMR (400 MHz; CDCl₃): δ 8.01 (s,1H), 7.40 (m, 3H), 7.37 (d, 1H), 7.27 (bm, 3H), 7.18 (d, 2H), 6.86 (d, 1H), 6.66 (dd, 1H), 5.83 (s, 1H), 4.79 (d, 1H), 4.54 (d, 1H), 4.51 (d, 1H), 4.39 (d, 1H), 3.62 (s, 6H), 3.45 (q, 1H), 2.95 (s, 3H), 2.92 (s, 3H), 2.14 (s, 3H), 2.11 (s, 3H), 1.29 (d, 3H).

### Example 9

### Preparation of cis-2,6-dimethyl-N,N-dimethylpiperidinium salt of esomeprazole

Esomeprazole sodium salt (0.368 g (1 mmol)) was added to a mixture of cis-2,6-dimethyl-N,N-dimethylpiperidinium iodide (0.270 g (0.5 mmol)), potassium carbonate (anhydrous) (1 g (7.3 mmol)) and water (1 ml). Dichloromethane (8ml) was added and the mixture was shaken (1 min). After separation, the organic phase was dried over Na₂SO₄ and filtered. The filtrate was concentrated to drieness at reduced pressure. 0.470 g (0.96 mmol) cis-2,6-dimethyl-N,N-dimethylpiperidinium salt of esomeprazole (amorphous foam) was obtained.

¹H-NMR (400 MHz; CDCl₃): δ 7.90 (s, 1H), 7.43 (d, 1H), 7.04 (d, 1H), 6.66 (dd, 1H), 4.61 (d, 1H), 4.44 (d, 1H), 3.68 (s, 3H), 3.62 (s, 3H), 3.41 (bm, 2H), 2.84 (s, 3H), 2.41 (s, 3H), 2.13 (s, 3H), 2.00 (s, 3H), 1.58 (m, 4H), 1.48 (m, 2H), 1.15 (dd, 6H).

## Claims

1. A process for preparation of a quaternary ammonium salt of (*S*)-5-methoxy-2-[[(4-methoxy-3,5-dimethyl-2-pyridinyl)-methyl]sulfinyl]-1*H-*benzimidazole (esomeprazole), 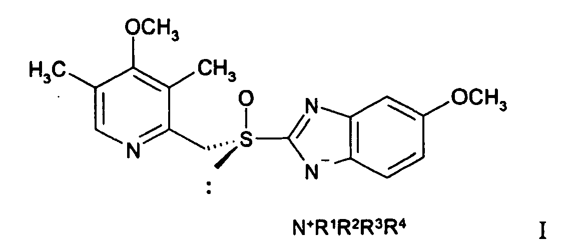 wherein
R₁, R₂, R₃ and R₄ are individually selected from:
(A) C₁-C₁₄alkyl group (which alkyl group is optionally substituted by one or more groups selected from amino, hydroxy, halogen, R₅O-, C₃-C₁₂ cycloalkyl (which cycloalkyl is optionally substituted by one or more groups selected from C₁-C₃ alkyl, hydroxy, C₁-C₃ alkoxy, halogen, oxo, R₂₃ₐOC(O)-, (R_{23b})(R_{23c})NC(O)-, R_{23d}C(O)N(R₂₃ₑ)-, R_{23f}(O)O-, R_{23g}OC(O)-NH-, (R₂₃ₕ)(R₂₃ⱼ)NC(O)O-), aryl or Het¹ (both groups optionally substituted by one to three groups selected from C₁₋C₇ alkyl, hydroxy, -CH₂OH, halogen, oxo, nitro, C₁-C₇ alkoxy, R₂₄ₐOC(O)-, (R_{24b})(R_{24c})NC(O)-, R_{24d}C(O)_{N}(R₂₄ₑ)-, R_{24f}C(O)O-, R_{24g}OC(O)-NH-, (R₂₄ₕ)(R₂₄ⱼ)NC(O)O-, aryl, Het³ or R₂₅C(O)- (which aryl and Het³ are optionally substituted by one or two halogens, C₁-C₄ alkyl, hydroxy C₁-C₄alkyl, C₁-C₄ alkoxy, hydroxy C₁-C₄ alkoxy, nitro)); R₆ -O-(CH₂)ₘ, -O-, R₇ₐOC(O)-, (R_{7b})(R_{7c})NC(O)-, R_{7d}C(O)N(R₇ₑ)-, R_{7f}C(O)O-, R_{7g}C(O)S-, R₇ₕOC(O)N(R₇ⱼ)-, (R₇ₖ)(R₇ₗ)NC(O)O-, R₇ₘOC(O)O-, R₈-SO₂-NH-, phtalimido, succinimido, R₉C(O)-, R₁₀-(CH₂)ₙ- C(O)-, (R₁₁ₐ)(R_{11b})(R_{11c})C-C(O)O-);
(B) aryl or Het² (both groups are optionally substituted by one to three groups selected from C₁-C₇ alkyl, hydroxy, C₁-C₇ alkoxy, halogen, R₁₂ₐOC(O)-, (R_{12b})(R_{12c})NC(O)-,
R_{12d}C(O)N(R₁₂ₑ)-, R_{12f}C(O)O-, R_{12g}OC(O)NR₁₂ₕ-, (R₁₂ⱼ)(R₁₂ₖ)NC(O)O-, aryl, benzoyl or Het⁴), R₁₃C(O)-, (R₁₄ₐ)(R_{14b})N-);
or R₁ and R₂ together may represent a cyclic structure containing 5-14 members, optionally substituted by one or more groups selected from hydroxy, oxo, C₁-C₇ alkyl (which alkyl group is optionally substituted by one or more groups selected from hydroxy, halogen, aryl or
Het⁷), R₁₅O-, R₁₆ₐOC(O)-, (R_{16b})(R_{16c})NC(O)-, R_{16d}C(O)N(R₁₆ₑ)-, R_{16f}(O)O-, R_{16g}OC(O)NR₁₆ₕ-, (R₁₆ⱼ)(R₁₆ₖ)NC(O)O-, R₁₇C(O)-, aryl or Het⁵ (which aryl and Het⁵ are optionally substituted by one or more of C₁-C₇ alkyl, hydroxy, oxo, C₁-C₇ alkoxy, halogen, R₂₆ₐOC(O)- , (R_{26b})(R_{26c})NC(O)-, R_{26d}C(O)N(R₂₆ₑ)-, R_{26f}C(O)O-, R_{26g}OC(O)NH-, (R₂₆ₕ)(R₂₆ⱼ)NC(O)O-, phenyl or benzoyl (which phenyl or benzoyl are optionally substituted by one or two halogens, C₁-C₅alkyl C(O)O-)), phtalimido, succinimido or (R₁₈ₐ)(R_{18b})(R_{18c})C-C(O)O-;
or R₁, R₂ and R₃ together may represent a cyclic structure containing 5-16 members, optionally substituted by one or more groups selected from hydroxy, oxo, C₁-C₇ alkyl (which alkyl group is optionally substituted by one or more groups selected from hydroxy, halogen, oxo, aryl or Het⁸), R₁₉O-, R₂₀C(O)-, aryl and Het⁶ (which aryl and Het⁶ are optionally substituted by one to three groups selected from C₁-C₇ alkyl, hydroxy, C₁-C₇ alkoxy, halogen, oxo, R₂₇ₐOC(O)- , (R_{27b})(R_{27c})NC(O)-, R_{27d}C(O)N(R₂₇ₑ)-, R_{27f}C(O)O-, R_{27g}OC(O)-NH, (R₂₇ₕ)(R₂₇ⱼ)NC(O)O-, phenyl or benzoyl), R₂₁ₐOC(O)- , (R_{21b})(R_{21c})NC(O)-, R_{21d}C(O)N(R₂₁ₑ)-, R_{21f}C(O)O-, R_{21g}OC(O)-NR₂₁ₕ-, (R₂₁ⱼ)(R₂₁ₖ)NC(O)O-, phtalimido, succinimido or (R₂₂ₐ)(R_{22b})(R_{22c}) C-C(O)O-;
R₅ is selected from C₁-C₆ alkyl, aryl, Het⁹ (which groups are optionally substituted by one or more groups selected from hydroxy, halogen, C₁-C₆ alkoxy);
R₆ is selected from aryl or Het¹⁰ (both groups optionally substituted by one or more groups selected from C₁-C₈ alkyl, hydroxy, C₁-C₇ alkoxy, halogen, R₂₈ₐOC(O)-, (R_{28b})(R_{28c})NC(O), R_{28d}C(O)N(R₂₈ₑ)-, R_{28f}C(O)O-, R_{28g}OC(O)-NH-, (R₂₈ₕ)(R₂₈ⱼ)NC(O)O- , aryl, benzoyl or Het¹¹);
R₇ₐ to R₇ₘ are independently selected, at each occurrence, from hydrogen, C₁-C₇ alkyl, aryl or Het¹² (which C₁-C₇ alkyl, aryl and Het¹² are optionally substituted by one or more groups selected from C₁-C₆alkyl, hydroxy, C₁-C₃ alkoxy, halogen, R₂₉ₐOC(O)- , (R_{29b})(R_{29c})NC(O)-, R_{29d}C(O)N(R₂₉ₑ)-, R_{29f}C(O)O-, R_{29g}OC(O)-NH-, (R₂₉ₕ)(R₂₉ⱼ)NC(O)O-, aryl, benzoyl or Het¹³);
R₈ is selected from C₁-C₆ alkyl, aryl or Het¹⁴ (which groups are optionally substituted by one or more groups selected from C₁-C₆ alkyl);
R₉ is selected from linear or branched C₁-C₁₂alkyl (optionally substituted by R₃₀OC(O)-), C₃-C₁₂ cycloalkyl (which cycloalkyl group is optionally further substituted by one or more groups selected from C₁-C₃ alkyl, hydroxy, C₁-C₃ alkoxy, halogen, R₃₁ₐOC(O)-, (R_{31b})(R_{31c})NC(O)-, R_{31d}C(O)NR₃₁ₑ-, R_{31f}C(O)O-, R_{31g}C(O)N(R₃₁ₕ)-, (R₃₁ⱼ)(R₃₁ₖ)NC(O)O-), aryl, benzoyl or Het¹⁵), aryl or Het¹⁶ (which aryl and Het¹⁶ are optionally substituted by one to three of the groups selected from C₁-C₆alkyl, hydroxy, C₁-C₃ alkoxy, ethylenedioxy, halogen, R₃₂ₐOC(O)-, (R_{32b})(R_{32c})NC(O)-, R_{32d}C(O)NR₃₂ₑ-, R_{32f}C(O)O-, R_{32g}OC(O)NH-, (R₃₂ₕ)(R₃₂ⱼ)NC(O)O-), aryl, benzoyl or Het¹⁷);
R₁₀ is selected from aryl and Het¹⁸ (which groups are optionally substituted by one to three groups selected from C₁-C₃ alkyl, hydroxy, C₁-C₃ alkoxy, halogen, -COOH, ethylenedioxy);
R₁₁ₐ is selected from hydroxy or -CH₂OH;
R_{11b} is phenyl (optionally substituted by one to three groups selected from C₁-C₃ alkyl, hydroxy, C₁-C₃ alkoxy, halogen, R₃₃ₐOC(O)- , (R_{33b})(R_{33c})NC(O)-, R_{33d}C(O)N(R₃₃ₑ)-, R_{33f}C(O)O-, R_{33g}OC(O)-NH-, (R₃₃ₕ)(R₃₃ⱼ)NC(O)O-);
R_{11c} is selected from hydrogen, C₅-C₆ cycloalkyl, phenyl (which groups are optionally substituted by one to three groups selected from C₁-C₃alkyl, hydroxy, C₁-C₃alkoxy, halogen, R₃₄ₐOC(O)- , (R_{34b})(R_{34c})NC(O)-, R_{34d}C(O)N(R₃₄ₑ)-, R_{34f}C(O)O- , R_{34g}OC(O)NH-, (R₃₄ₕ)(R₃₄ⱼ)NC(O)O-);
R₁₂ₐ to R₁₂ₖ are independently selected, at each occurrence, from hydrogen, C₁-C₇alkyl, aryl, Het¹⁹ (which groups are optionally substituted by one or more groups selected from C₁-C₆alkyl, hydroxy, C₁-C₃alkoxy, halogen, R₃₅ₐOC(O)-, (R_{35b})(R_{35c})NC(O)-, R_{35d}C(O)N(R₃₅ₑ)-, R_{35f}C(O)O-, R_{35g}OC(O)-NH-, (R₃₅ₕ)(R₃₅ⱼ)NC(O)O-, aryl, benzoyl or Het²⁰);
R₁₃ is selected from hydrogen or C₁-C₆ alkyl;
R₁₄ₐ to R_{14b} are independently selected, at each occurrence, from hydrogen or C₁-C₆ alkyl;
R₁₅ is selected from C₁-C₆ alkyl, aryl or Het²¹ (which groups are optionally substituted by one or more groups selected from hydroxy, halogen or C₁-C₆ alkoxy);
R₁₆ₐ to R₁₆ₖ are independently selected from, at each occurrence, hydrogen, C₁-C₇ alkyl, aryl or Het²² (which groups are optionally substituted by one or more groups selected from C₁-C₆ alkyl, hydroxy, C₁-C₃ alkoxy, halogen, R₃₆ₐOC(O)- , (R_{36b})(R_{36c})NC(O)-, R_{36d}C(O)N(R₃₆ₑ)-, R_{36f}C(O)O- , R_{36g}OC(O)-NH-, (R₃₆ₕ)(R₃₆ⱼ)NC(O)O-, aryl, benzoyl or
Het²³;
R₁₇ is selected from hydrogen or C₁-C₆ alkyl;
R₁₈ₐ is selected from hydroxy or -CH₂OH;
R_{18b} is phenyl (optionally substituted by one to three groups selected from C₁-C₃ alkyl, hydroxy, C₁-C₃ alkoxy, halogen, R₃₇ₐOC(O)-, (R_{37b})(R_{37c})NC(O)-, R_{37d}C(O)N(R₃₇ₑ)-, R^{37f}C(O)O-, R_{37g}OC(O)-NH-, (R₃₇ₕ)(R₃₇ⱼ)NC(O)O-);
R_{18c} is selected from hydrogen, C₅-C₆ cycloalkyl or phenyl (which optionally is substituted by one to three groups selected from C₁-C₃ alkyl, hydroxy, C₁-C₃ alkoxy, halogen, R₃₈ₐOC(O)-, (R_{38b})(R_{38c})NC(O)-, R_{38d}C(O)N(R₃₈ₑ)-, R_{38f}C(O)O-, R_{38g}OC(O)-NH-, (R₃₈ₕ)(R₃₈ⱼ)NC(O)O-);
R₁₉ is selected from C₁-C₆ alkyl, aryl or Het²⁴ (which groups are optionally substituted by one or more groups selected from hydroxy, halogen or C₁-C₆alkoxy);
R₂₀ is selected from hydrogen and C₁-C₆alkyl;
R₂₁ₐ to R₂₁ₖ are independently selected, at each occurence, from
hydrogen, C₁-C₇ alkyl, aryl or Het²⁵ (which groups are optionally substituted by one or more groups selected from C₁-C₆alkyl, hydroxy, C₁-C₃ alkoxy, halogen, R₃₉ₐOC(O)- , (R_{39b})(R_{39c})NC(O)-, R_{39d}C(O)N(R₃₉ₑ)-, R_{39f}C(O)O- , R_{39g}OC(O)-NH-, (R₃₉ₕ)(R₃₉ⱼ)NC(O)O-, aryl, benzoyl or Het²⁶);
R₂₂ₐ is selected from hydroxy or -CH₂OH;
R_{22b} is phenyl (optionally substituted by one to three groups selected from C₁-C₃ alkyl, hydroxy, C₁-C₃ alkoxy, halogen, R₄₀ₐOC(O)- , (R_{40b})(R_{40c})NC(O)-, R_{40d}C(O)N(R₄₀ₑ)-, R_{40f}C(O)O-, R_{40g}OC(O)NH-, (R₄₀ₕ)(R₄₀ⱼ)NC(O)O-);
R_{22c} is selected from hydrogen, C₅-C₆ cycloalkyl or phenyl (which optionally is substituted by one or three groups selected from C₁-C₃ alkyl, hydroxy, C₁-C₃ alkoxy, halogen, R₄₁ₐOC(O)-, (R_{41b})(R_{41c})NC(O)-, R_{41d}C(O)N(R₄₁ₑ)-, R_{41f}C(O)O-, R_{41g}OC(O)NH-, (R₄₁ₕ)(R₄₁ⱼ)NC(O)O-);
R₂₃ₐ to R₂₃ⱼ are independently selected, at each occurrence, from hydrogen or C₁-C₆alkyl;
R₂₄ₐ to R₂₄ⱼ are independently selected, at each occurrence, from hydrogen or C₁-C₆alkyl;
R₂₅ is selected from C₁-C₄alkyl, aryl or Het²⁷ (which aryl and Het²⁷ are optionally substituted by one or two halogens, C₁-C₄ alkyl, hydroxy C₁-C₄alkyl, C₁-C₄ alkoxy, hydroxy C₁-C₄ alkoxy, nitro);
R₂₆ₐ to R₂₆ⱼ are independently selected, at each occurrence, from hydrogen or C₁-C₆alkyl;
R₂₇ₐ to R₂₇ⱼ are independently selected, at each occurrence, from hydrogen or C₁-C₆alkyl;
R₂₈ₐ to R₂₈ⱼ are independently selected, at each occurrence, from hydrogen or C₁-C₆alkyl;
R₂₉ₐ to R₂₉ⱼ are independently selected, at each occurrence, from hydrogen or C₁-C₆alkyl;
R₃₀ is selected from hydrogen or C₁-C₆ alkyl;
R₃₁ₐ to R₃₁ₖ are independently selected, at each occurrence, from hydrogen or C₁-C₆alkyl;
R₃₂ₐ to R₃₂ⱼ are independently selected, at each occurrence, from hydrogen or C₁-C₆alkyl;
R₃₃ₐ to R₃₃ⱼ are independently selected, at each occurrence, from hydrogen or C₁-C₆alkyl;
R₃₄ₐ to R₃₄ⱼ are independently selected, at each occurrence, from hydrogen or C₁-C₆alkyl;
R₃₅ₐ to R₃₅ⱼ are independently selected, at each occurrence, from hydrogen or C₁-C₆alkyl;
R₃₆ₐ to R₃₆ⱼ are independently selected, at each occurrence, from hydrogen or C₁-C₆alkyl;
R₃₇ₐ to R₃₇ⱼ are independently selected, at each occurrence, from hydrogen or C₁-C₆alkyl;
R₃₈ₐ to R₃₈ⱼ are independently selected, at each occurrence, from hydrogen or C₁-C₆alkyl;
R₃₉ₐ to R₃₉ⱼ are independently selected, at each occurrence, from hydrogen or C₁-C₆alkyl;
R₄₀ₐ to R₄₀ⱼ are independently selected, at each occurrence, from hydrogen or C₁-C₆alkyl;
R₄₁ₐ to R₄₁ⱼ are independently selected, at each occurrence, from hydrogen or C₁-C₆ alkyl;
Het groups (Het¹ to Het²⁷) include those ring systems having a total number of atoms in the ring system between five and twelwe atoms and containing 1 to 5 heteroatoms (selected from N, O and S)
m is an integer selected from 1 to 5;
n is an integer selected from 1 to 3.
which process comprises the following steps:
(i): mixing esomeprazole and N⁺(R₁)(R₂)(R₃)(R₄)X⁻;
wherein
R₁, R₂, R₃ and R₄ are as defined above;
X⁻ is selected from Cl⁻, Br⁻, I⁻, C₁-C₆ alkyl carboxylates, C₁-C₆ alkyl sulphonates, HSO₄⁻ and OH ;
in an aqueous solvent system comprising more than 40 % w/w potassium carbonate;
(ii): adding a water immiscible chlorinated hydrocarbon solvent;
(iii): isolating the organic phase;
(iv): recovering of the compound of formula I.

2. A process according to claim 1 wherein the aqueous solvent system in step (i) comprises more than 50 % by weight potassium carbonate.

3. A process according to claim 1 wherein the aqueous solvent system in step (i) is saturated with potassium carbonate.

4. A process according to claim 1 wherein the chlorinated water immiscible solvent is selected from 1,2 dichlormethane, trichlormethane and 1,2-dichloretane.

5. A process according to claim 1 wherein esomeprazole in step (i) is the sodium salt or potassium salt of esomeprazole.

6. A process according to any of claims 1 and 5 wherein R₂, R₃ and R₄ are individually selected from, at each occurrence, linear or branched C₁-C₆ alkyl group.

7. A process according to claim 1 wherein R₁ is selected from linear or branched C₁-C₈ alkyl group (which alkyl group is optionally substituted by one or more groups selected from amino, hydroxy, halogen, R₅O- or aryl); R₂, R₃ and R₄ are individually selected from linear or branched C₁-C₄alkyl group (which alkyl group is optionally substituted by one or more groups selected from amino, hydroxy, halogen or R₅O-) or aryl.

8. A process according to claim 1 wherein R₁ is selected from linear or branched C₁-C₈ alkyl group, which alkyl group is optionally substituted by one or more groups selected from amino, hydroxy, halogen, R₅O- or phenyl; R₂, R₃ and R₄ are independently, at each occurrence, selected from methyl, ethyl, n-propyl or isopropyl (which group is optionally substituted by one or more groups selected from amino, hydroxy, halogen or R₅O-) or phenyl.

9. A process according to claim 1 wherein R₁ and R₂ together may represent a cyclic structure containing 5 to 10 members, optionally substituted by on or more groups selected linear or branched C₁-C₅ alkyl group, amino, hydroxy, halogen or R₅O-; R₃ and R₄ are selected from linear or branched C₁-C₄ alkyl group.

## Patentansprüche

1. Verfahren zur Herstellung eines quartären Ammoniumsalzes von (S)-5-Methoxy-2-[[(4-methoxy-3,5-dimethyl-2-pyridinyl)-methyl]sulfinyl]-1H-benzimidazol (Esomeprazol), 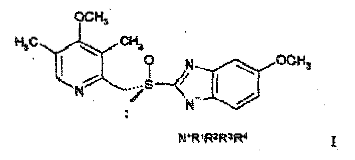 worin
R₁, R₂, R₃ und R₄ individuell ausgewählt sind unter:
(A) einer C₁-C₁₄-Alkylgruppe (die gegebenenfalls durch eine oder mehrere unter Amino, Hydroxy, Halogen, R₅O-, C₃-C₁₂-Cycloalkyl (das gegebenenfalls durch eine oder mehrere unter C₁-C₃-Alkyl, Hydroxy, C₁-C₃-Alkoxy, Halogen, Oxo, R₂₃ₐOC(O)-, (R_{23b}) (R_{23c})NC(O) -, R_{23d}C(O)N(R₂₃ₑ) -, R_{23f}C(O)O-, R_{23g}OC(O) -NH-, (R₃₃ₕ) (R₂₃ⱼ) NC (O) O- ausgewählte Gruppen substituiert ist), Aryl oder Het¹ (wobei beide Gruppen gegebenenfalls durch eine bis drei unter C₁-C₇-Alkyl, Hydroxy, -CH₂OH, Halogen, Oxo, Nitro, C₁-C₇-Alkoxy, R₂₄ₐOC(O)-, (R_{24b})(R_{24c}) NC (O)-, R_{24d}C(O) N(R₂₄ₑ)-, R_{24f}C(O)O-, R_{24g}OC(O) -NH-, (R₂₄ₕ)(R₂₄ⱼ)NC(O)O-, Aryl, Het³ oder R₂₅C(O)- (wobei Aryl und Het³ gegebenenfalls durch ein oder zwei Halogene, C₁-C₄-Alkyl, Hydroxy-C₁-C₄-alkyl, C₁-C₄-Alkoxy, Hydroxy-C₁-C₄-alkoxy, Nitro substituiert sind) ausgewählte Gruppen substituiert sind), R₆-O-(CH₂)ₘ-O-, R₇ₐOC(O)-, (R_{7b}) (R_{7c})NC(O)-, R_{7d}C(O)N(R₇ₑ)-, R_{7f}C(O)O-, R_{7g}C(O)S-, R₇ₕOC(O)N(R₇ⱼ)-, (R₇ₖ) (R₇₁)NC(O)O-, R₇ₘOC(O)O-, R₈-SO₂-NH-, Phthalimido, Succinimido, R₉C(O)-, R₁₀-(CH₂)ₙ-C(O)-, (R₁₁ₐ) (R_{11b}) (R_{11c})C-C(O)O- ausgewählte Gruppen substituiert ist);
(B) Aryl oder Het² (wobei beide Gruppen gegebenenfalls durch eine bis drei unter C₁-C₇-Alkyl, Hydroxy, C₁-C₇-Alkoxy, Halogen, R₁₂ₐOC(O)-, (R_{12b}) (R_{12c})NC(O)-, R_{12d}C(O)N(R₁₂ₑ)-, R_{12f}C(O)O-, R_{12g}OC(O)NR₁₂ₕ-, (R₁₂ⱼ)(R₁₂ₖ)NC(O)O-, Aryl, Benzoyl oder Het⁴, R₁₃C(O)-, (R₁₄ₐ)(R_{14b})N- ausgewählte Gruppen substituiert sind);
oder R₁ und R₂ zusammen für eine cyclische Struktur mit 5-14 Gliedern, die gegebenenfalls durch eine oder mehrere unter Hydroxy, Oxo, C₁-C₇-Alkyl (das gegebenenfalls durch eine oder mehrere unter Hydroxy, Halogen, Aryl oder Het⁷ ausgewählte Gruppen substituiert ist), R₁₅O-, R₁₆ₐOC(O)-, (R_{16b})(R_{16c})NC(O)-, R_{16d}C(O)N(R₁₆ₑ)-, R_{16f}C(O)O-, R_{16g}OC(O)NR₁₆ₕ-, (R₁₆ⱼ) (R₁₆ₖ)NC(O)O-, R₁₇C(O)-, Aryl oder Het⁵ (wobei Aryl und Het⁵ gegebenenfalls ein- oder mehrfach durch C₁-C₇-Alkyl, Hydroxy, Oxo, C₁-C₇-Alkoxy, Halogen, R₂₆ₐOC(O)-, (R_{26b}) (R_{26c})NC(O)-, R_{26d}C(O)N(R₂₆ₑ)-, R_{26f}C(O)O-, R_{26g}OC(O)NH-, (R₂₆ₕ)(R₂₆ⱼ)NC(O)O-, Phenyl oder Benzoyl (wobei Phenyl oder Benzoyl gegebenenfalls ein- oder zweifach durch Halogen, C₁-C₅-Alkyl-C(O)O-substituiert sind) substituiert sind), Phthalimido, Succinimido oder (R₁₈ₐ)(R_{18b})(R_{18c})C-C(O)O- ausgewählte Gruppen substituiert ist, stehen können;
oder R₁, R₂ und R₃ zusammen für eine cyclische Struktur mit 5-16 Gliedern, die gegebenenfalls durch eine oder mehrere unter Hydroxy, Oxo, C₁-C₇-Alkyl (das gegebenenfalls durch eine oder mehrere unter Hydroxy, Halogen, Oxo, Aryl oder Het⁸ ausgewählte Gruppen substituiert ist), R₁₉O-, R₂₀C(O)- Aryl und Het⁶ (wobei Aryl und Het⁶ gegebenenfalls durch eine bis drei unter C₁-C₇-Alkyl, Hydroxy, C₁-C₇-Alkoxy, Halogen, Oxo, R₂₇ₐOC(O)-, (R_{27b})(R_{27c})NC(O)-, R₂₇ₐC(O)N(R₂₇ₑ)-, R_{27f}C(O)O-, R_{27g}OC(O)-NH-, (R₂₇ₕ)(R₂₇ⱼ)NC(O)O-, Phenyl oder Benzoyl ausgewählte Gruppen substituiert sind), R₂₁ₐOC(O)-, (R_{21b})(R_{21c})NC(O)-, R_{21d}C(O)N (R₂₁ₑ)-, R_{21f}C(O)O-, R_{21g}OC(O)-NR₂₁ₕ-, (R₂₁ⱼ)(R₂₁ₖ)NC(O)O-, Phthalimido, Succinimido oder (R₂₂ₐ)(R_{22b})(R_{22c})C-C(O)O- ausgewählte Gruppen substituiert ist, stehen können;
R₅ unter C₁-C₆-Alkyl, Aryl, Het⁹ (wobei diese Gruppen gegebenenfalls durch eine oder mehrere unter Hydroxy, Halogen, C₁-C₆-Alkoxy ausgewählte Gruppen substituiert sind) ausgewählt ist;
R₆ unter Aryl oder Het¹⁰ (wobei beide Gruppen gegebenenfalls durch eine oder mehrere unter C₁-C₈-Alkyl, Hydroxy, C₁-C₇-Alkoxy, Halogen, R₂₈ₐOC(O)-, (R_{28b})(R_{28c})NC(O)-, R_{28d}C(O)N(R₂₈ₑ)-, R_{28f}C(O)O-, R_{28g}OC(O)-NH-, (R₂₈ₕ)(R₂₈ⱼ)NC(O)O-, Aryl, Benzoyl oder Het¹¹ ausgewählte Gruppen substituiert sind) ausgewählt ist;
R₇ₐ bis R₇ₘ jeweils unabhängig voneinander unter Wasserstoff, C₁-C₇-Alkyl, Aryl oder Het¹² (wobei C₁-C₇-Alkyl, Aryl und Het¹² gegebenenfalls durch eine oder mehrere unter C₁-C₆-Alkyl, Hydroxy, C₁-C₃-Alkoxy, Halogen, R₂₉ₐOC(O)-, (R_{29b})(R_{29c})NC(O)-, R_{29d}C(O)N(R₂₉ₑ) -, R_{29f}C(O)O-, R_{29g}OC(O)-NH-, (R₂₉ₕ)(R₂₉ⱼ)NC(O)O-, Aryl, Benzoyl oder Het¹³ ausgewählte Gruppen substituiert sind) ausgewählt sind;
R₈ unter C₁-C₆-Alkyl, Aryl oder Het¹⁴ (wobei diese Gruppen gegebenenfalls durch eine oder mehrere unter C₁-C₆-Alkyl ausgewählte Gruppen substituiert sind) ausgewählt ist;
R₉ unter linearem oder verzweigtem C₁-C₁₂-Alkyl- (das gegebenenfalls durch R₃₀OC(O)- substituiert ist), C₃-C₁₂-Cycloalkyl (das gegebenenfalls ferner durch eine oder mehrere unter C₁-C₃-Alkyl, Hydroxy, C₁-C₃-Alkoxy, Halogen, R₃₁ₐOC(O)-, (R_{31b})(R_{31c})NC(O)-, R₃₁ₐC(O)NR₃₁ₑ-, R_{31f}C(O)O-, R_{31g}C(O)N(R₃₁ₕ)-, (R₃₁ⱼ)(R₃₁ₖ)NC(O)O-, Aryl, Benzoyl oder Het¹⁵ ausgewählte Gruppen substituiert ist), Aryl oder Het¹⁶ (wobei Aryl und Het¹⁶ gegebenenfalls durch eine bis drei unter C₁-C₆-Alkyl, Hydroxy, C₁-C₃-Alkoxy, Ethylendioxy, Halogen, R₃₂ₐOC(O)-, (R_{32b})(R_{32c}) NC (O)-, R_{32d}C(O)NR₃₂ₑ-, R_{32f}C(O)O-, R_{32g}OC(O)NH-, (R₃₂ₕ)(R₃₂ⱼ)NC(O)O-, Aryl, Benzoyl oder Het²⁷ ausgewählte Gruppen substituiert sind) ausgewählt ist;
R₁₀ unter Aryl und Het¹⁸ (wobei diese Gruppen gegebenenfalls durch eine bis drei unter C₁-C₃-Alkyl, Hydroxy, C₁-C₃-Alkoxy, Halogen, -COOH, Ethylendioxy ausgewählte Gruppen substituiert sind) ausgewählt ist;
R₁₁ₐ unter Hydroxy oder -CH₂OH ausgewählt ist;
R_{11b} für Phenyl (das gegebenenfalls durch eine bis drei unter C₁-C₃-Alkyl, Hydroxy, C₁-C₃-Alkoxy, Halogen, R₃₃ₐOC(O)-, (R_{33b})(R_{33c})NC(O)-, R_{33d}C(O)N(R₃₃ₑ)-, R_{33f}C(O)O-, R_{33g}OC(O)-NH-, (R₃₃ₕ)(R₃₃ⱼ)NC(C)O- ausgewählte Gruppen substituiert ist) steht;
R_{11c} unter Wasserstoff, C₅-C₆-Cycloalkyl, Phenyl (wobei diese Gruppen gegebenenfalls durch eine bis drei unter C₁-C₃-Alkyl, Hydroxy, C₁-C₃-Alkoxy, Halogen, R₃₄ₐOC(O)-, (R_{34b})(R_{34c})NC(O)-, R_{34d}C(O)N(R₃₄ₑ)-, R_{34f}C(O)O-, R_{34g}OC(O)NH-, (R₃₄ₕ)(R₃₄ⱼ)NC(O)O- ausgewählte Gruppen substituiert sind) ausgewählt ist;
R₁₂ₐ bis R₁₂ₖ jeweils unabhängig voneinander unter Wasserstoff, C₁-C₇-Alkyl, Aryl, Het¹⁹ (wobei diese Gruppen gegebenenfalls durch eine oder mehrere unter C₁-C₆-Alkyl, Hydroxy, C₁-C₃-Alkoxy, Halogen, R₃₅ₐOC(O)-, (R_{35b})(R_{35c})NC(O)-, R_{35d}C(O)N(R₃₅ₑ)-, R_{35f}C(O)O-, R_{35g}OC(O)-NH-, (R₃₅ₕ)(R₃₅ⱼ)NC(O)O-, Aryl, Benzoyl oder Het²⁰ ausgewählte Gruppen substituiert sind) ausgewählt sind;
R₁₃ unter Wasserstoff oder C₁-C₆-Alkyl ausgewählt ist;
R₁₄ₐ bis R_{14b} jeweils unabhängig voneinander unter Wasserstoff oder C₁-C₆-Alkyl ausgewählt sind;
R₁₅ unter C₁-C₆-Alkyl, Aryl oder Het²¹ (wobei diese Gruppen gegebenenfalls durch eine oder mehrere unter Hydroxy, Halogen oder C₁-C₆-Alkoxy ausgewählte Gruppen substituiert sind) ausgewählt ist;
R₁₆ₐ bis R₁₆ₖ jeweils unabhängig voneinander unter Wasserstoff, C₁-C₇-Alkyl, Aryl oder Het²² (wobei diese Gruppen gegebenenfalls durch eine oder mehrere unter C₁-C₆-Alkyl, Hydroxy, C₁-C₃-Alkoxy, Halogen, R₃₆ₐOC(O)-, (R_{36b})(R_{36c})NC(C)-, R_{36d}C(O)N(R₃₆ₑ)-, R_{36f}C(O)O-, R_{36g}OC(O)-NH-, (R₃₆ₕ)(R₃₆ⱼ)NC(O)O-, Aryl, Benzoyl oder Het²³ ausgewählte Gruppen substituiert sind) ausgewählt sind;
R₁₇ unter Wasserstoff oder C₁-C₆-Alkyl ausgewählt ist;
R₁₈ₐ unter Hydroxy oder -CH₂OH ausgewählt ist;
R_{18b} für Phenyl (das gegebenenfalls durch eine bis drei unter C₁-C₃-Alkyl, Hydroxy, C₁-C₃-Alkoxy, Halogen, R₃₇ₐOC(O)-, (R_{37b}) (R_{37c})NC(O)-, R_{37d}C(O)N(R₃₇ₑ) -, R_{37f}C(O)O-, R_{37g}OC(O) -NH-, (R₃₇ₕ)(R₃₇ⱼ)NC(O)O- ausgewählte Gruppen substituiert ist) steht;
R_{18c} unter Wasserstoff, C₅-C₆-Cycloalkyl oder Phenyl (wobei diese Gruppen gegebenenfalls durch eine bis drei unter C₁-C₃-Alkyl, Hydroxy, C₁-C₃-Alkoxy, Halogen, R₃₈ₐOC(O)-, (R_{32b}) (R₃₈ₑ)NC(O)-, R_{38d}C(O)N(R₃₈ₑ)-, R_{30f}C(O)O-, R_{38g}OC(O)-NH-, (R₃₈ₕ)(R₃₈ⱼ)NC(O)O- ausgewählte Gruppen substituiert sind) ausgewählt ist;
R₁₉ unter C₁-C₆-Alkyl, Aryl oder Het²⁴ (wobei diese Gruppen gegebenenfalls durch eine oder mehrere unter Hydroxy, Halogen oder C₁-C₆-Alkoxy ausgewählte Gruppen substituiert sind) ausgewählt ist;
R₂₀ unter Wasserstoff oder C₁-C₆-Alkyl ausgewählt ist;
R₂₁ₐ bis R₂₁ₖ jeweils unabhängig voneinander unter Wasserstoff, C₁-C₇-Alkyl, Aryl oder Het²⁵ (wobei diese Gruppen gegebenenfalls durch eine oder mehrere unter C₁-C₆-Alkyl, Hydroxy, C₁-C₃-Alkoxy, Halogen, R₃₉ₐOC(O)-, (R_{39b})(R_{39c})NC(O)-, R_{39d}C(O)N(R₃₉ₑ)-, R_{39f}C(O)O-, R_{39g}OC(O)-NH-, (R₃₉ₕ)(R₃₉ⱼ) NC(O)O-, Aryl, Benzoyl oder Het²⁶ ausgewählte Gruppen substituiert sind) ausgewählt sind;
R₂₂ₐ unter Hydroxy oder -CH₂OH ausgewählt ist;
R_{22b} für Phenyl (das gegebenenfalls durch eine bis drei unter C₁-C₃-Alkyl, Hydroxy, C₁-C₃-Alkoxy, Halogen, R₄₀ₐOC(O)-, (R_{40b})(R_{40c})NC(O)-, R_{40d}C(O)N(R₄₀ₑ)-, R_{40f}C(O)O-, R_{40g}OC(O)NH-, (R₄₀ₕ)(R₄₀ⱼ)NC(O)O- ausgewählte Gruppen substituiert ist) steht;
R_{22c} unter Wasserstoff, C₅-C₆-Cycloalkyl, Phenyl (welches gegebenenfalls durch eine oder drei unter C₁-C₃-Alkyl, Hydroxy, C₁-C₃-Alkoxy, Halogen, R₄₁ₐOC(O)-, (R_{41b})(R_{41c})NC(O)-, R_{41d}C(O)N(R₄₁ₑ)-, R_{41f}C(O)O-, R_{41g}OC(O)NH-, (R₄₁ₕ)(R₄₁ⱼ)NC(O)O-ausgewählte Gruppen substituiert ist) ausgewählt ist;
R₂₃ₐ bis R₂₃ⱼ jeweils unabhängig voneinander unter Wasserstoff oder C₁-C₆-Alkyl ausgewählt sind;
R₂₄ₐ bis R₂₄ⱼ jeweils unabhängig voneinander unter Wasserstoff oder C₁-C₆-Alkyl ausgewählt sind;
R₂₀ unter C₁-C₄-Alkyl, Aryl oder Het²⁷ (wobei Aryl und Het²⁷ gegebenenfalls ein- oder zweifach durch Halogen, C₁-C₄-Alkyl, Hydroxy-C₁-C₄-alkyl, C₁-C₄-Alkoxy, Hydroxy-C₁-C₄-alkoxy, Nitro subsitutiert sind) ausgewählt ist;
R₂₆ₐ bis R₂₆ⱼ jeweils unabhängig voneinander unter Wasserstoff oder C₁-C₆-Alkyl ausgewählt sind;
R₂₇ₐ bis R₂₇ⱼ jeweils unabhängig voneinander unter Wasserstoff oder C₁-C₆-Alkyl ausgewählt sind;
R₂₈ₐ bis R₂₈ⱼ jeweils unabhängig voneinander unter Wasserstoff oder C₁-C₆-Alkyl ausgewählt sind;
R₂₉ₐ bis R₂₉ⱼ jeweils unabhängig voneinander unter Wasserstoff oder C₁-C₆-Alkyl ausgewählt sind;
R₃₀ unter Wasserstoff oder C₁-C₆-Alkyl ausgewählte ist;
R₃₁ₐ bis R₃₁ₖ jeweils unabhängig voneinander unter Wasserstoff oder C₁-C₆-Alkyl ausgewählt sind;
R₃₂ₐ bis R₃₂ⱼ jeweils unabhängig voneinander unter Wasserstoff oder C₁-C₆-Alkyl ausgewählt sind;
R₃₃ₐ bis R₃₃ⱼ jeweils unabhängig voneinander unter Wasserstoff oder C₁-C₆-Alkyl ausgewählt sind;
R₃₄ₐ bis R₃₄ⱼ jeweils unabhängig voneinander unter Wasserstoff oder C₁-C₆-Alkyl ausgewählt sind;
R₃₅ₐ bis R₃₅ⱼ jeweils unabhängig voneinander unter Wasserstoff oder C₁-C₆-Alkyl ausgewählt sind;
R₃₆ₐ bis R₃₆ⱼ jeweils unabhängig voneinander unter Wasserstoff oder C₁-C₆-Alkyl ausgewählt sind;
R₃₇ₐ bis R₃₇ⱼ jeweils unabhängig voneinander unter Wasserstoff oder C₁-C₆-Alkyl ausgewählt sind;
R₃₈ₐ bis R₃₈ⱼ jeweils unabhängig voneinander unter Wasserstoff oder C₁-C₆-Alkyl ausgewählt sind;
R₃₉ₐ bis R₃₉ⱼ jeweils unabhängig voneinander unter Wasserstoff oder C₁-C₆-Alkyl ausgewählt sind;
R₄₀ₐ bis R₄₀ⱼ jeweils unabhängig voneinander unter Wasserstoff oder C₁-C₆-Alkyl ausgewählt sind;
R₄₁ₐ bis R₄₁ⱼ jeweils unabhängig voneinander unter Wasserstoff oder C₁-C₆-Alkyl ausgewählt sind;
Het-Gruppen (Het¹ bis Het²⁷) diejenigen Ringsysteme umfassen, die eine Gesamtzahl von Atomen im Ringsystem zwischen fünf und zwölf Atomen aufweisen und 1 bis 5 Heteroatome (die unter N, O und S ausgewählt sind) enthalten;
m für eine ganze Zahl von 1 bis 5 steht;
n für eine ganze Zahl von 1 bis 3 steht;
bei dem man:
(i) : Esomeprazol und N⁻(R₁) (R₂) (R₃) (R₄) X⁻;
worin
R₁, R₂, R₃ und R₄ die oben angegebene Bedeutung besitzen;
X⁻ unter Cl⁻, Br⁻, I⁻, C₁-C₆-Alkylcarboxylaten, C₁-C₆-Alkylsulfonaten, HSO₄⁻ und OH⁻ ausgewählt ist;
in einem wäßrigen Lösungsmittel System, das mehr als 40 Gew.-% Kaliumcarbonat enthält, mischt;
(ii) : ein mit Wasser nicht mischbares chloriertes Kohlenwasserstofflösungsmittel zugibt;
(iii): die organische Phase isoliert;
(iv): die Verbindung der Formel I gewinnt.

2. Verfahren nach Anspruch 1, bei dem das wäßrige Lösungsmittelsystem in Schritt (i) mehr als 50 Gew.-% Kaliumcarbonat enthält.

3. Verfahren nach Anspruch 1, bei dem das wäßrige Lösungsmittelsystem in Schritt (i) mit Kaliumcarbonat gesättigt ist.

4. Verfahren nach Anspruch 1, bei dem man das chlorierte, mit Wasser nicht mischbare Lösungsmittel unter 1,2-Dichlormethan, Trichlormethan und 1,2-Dichlorethan auswählt.

5. Verfahren nach Anspruch 1, bei dem es sich bei Esomeprazol in Schritt (i) um das Natriumsalz oder Kaliumsalz von Esomeprazol handelt.

6. Verfahren nach einem der Ansprüche 1 und 5, bei dem R₂, R₃ und R₄ jeweils individuell unter einer linearen oder verzweigten C₁-C₆-Alkylgruppe ausgewählt sind.

7. Verfahren nach Anspruch 1, bei dem R₁ unter einer linearen oder verzweigten C₁-C₈-Alkylgruppe (die gegebenenfalls durch eine oder mehrere unter Amino, Hydroxy, Halogen, R₅O- oder Aryl ausgewählte Gruppen substituiert ist) ausgewählt ist; R₂, R₃ und R₄ individuell unter einer linearen oder verzweigten C₁-C₄-Alkylgruppe (die gegebenenfalls durch eine oder mehrere unter Amino, Hydroxy, Halogen oder R₅O- ausgewählte Gruppen substituiert ist) oder Aryl ausgewählt sind.

8. Verfahren nach Anspruch 1, bei dem R₁ unter einer linearen oder verzweigten C₁-C₈-Alkylgruppe, die gegebenenfalls durch eine oder mehrere unter Amino, Hydroxy, Halogen, R₅O- oder Phenyl ausgewählte Gruppen substituiert ist, ausgewählt ist; R₂, R₃ und R₄ jeweils unabhängig voneinander unter Methyl, Ethyl, n-Propyl oder Isopropyl (das gegebenenfalls durch eine oder mehrere unter Amino, Hydroxy, Halogen oder R₅O- ausgewählte Gruppen substituiert ist) oder Phenyl ausgewählt sind.

9. Verfahren nach Anspruch 1, bei dem R₁ und R₂ zusammen für eine cyclische Struktur mit 5 bis 10 Gliedern, die gegebenenfalls durch eine oder mehrere unter einer linearen oder verzweigten C₁-C₅-Alkylgruppe, Amino, Hydroxy, Halogen oder R₅O-ausgewählten Gruppen substituiert ist, stehen können; R₃ und R₄ unter einer linearen oder verzweigten C₁-C₄-Alkylgruppe ausgewählt sind.

## Revendications

1. Procédé pour la préparation d'un sel d'ammonium quaternaire de (*S*)-5-méthoxy-2-[[(4-méthoxy-3,5-diméthyl-2-pyridinyl)-méthyl]sulfinyl]-1H-benzimidazole (ésoméprazole), 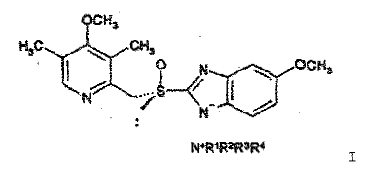 où
R₁, R₂, R₃ et R₄ sont individuellement choisis parmi :
(A) un groupe alkyle en C₁-C₁₄ (ledit groupe alkyle est facultativement substitué par un ou plusieurs groupes choisis parmi amino, hydroxy, halogène, R₅O-, un cycloalkyle en C₃-C₁₂ (ledit cycloalkyle est facultativement substitué par un ou plusieurs groupes choisis parmi alkyle en C₁-C₃, hydroxy, alcoxy en C₁-C₃, halogène, oxo, R₂₃ₐOC(O)-, (R_{23b}) (R_{23c})NC(O)-, R_{23d}C(O) N (R₂₃ₑ) - R_{23f}C (O) O-, R_{23g}OC(O)-NH-, (R₂₃ₕ) (R₂₃ⱼ)NC(O)O-), aryle ou Het¹ (les deux groupes facultativement substitués par un à trois groupes choisis parmi alkyle en C₁-C₇, hydroxy, -CH₂OH, halogène, oxo, nitro, alcoxy en C₁-C₇ R₂₄ₐOC(O)-, (R_{24b}) (R_{24c}) NC (O) -, R_{24d}C (O) N (R₂₄ₑ) -, R_{24f}C(O)O-, R_{24g}OC(O)-NH-, (R₂₄ₕ) (R₂₄ⱼ)NC(O)O-, aryle, Het³ ou R₂₅C(O)- (ledit aryle et Het³ sont facultativement substitués par un ou deux halogènes, alkyle en C₁-C₄, hydroxy(alkyle en C₁-C₄), alcoxy en C₁-C₄, hydroxy(alcoxy en C₁-C₄), nitro)) ; R₆-O- (CH₂)ₘ-O-, R₇ₐOC(O) -, (R_{7b})(R_{7c})NC(O) -, R_{7d}C(O)N(R₇ₐ)-, R_{7f}C(O)O-, R_{7g}C(O)S-, R₇ₕOC(O)N(R₇ⱼ)-, (R₇ₖ) (R₇₁) NC (O) O-, R₇ₘOC(O)O-, R₈SO₂-NH-, phtalimido, succinimido, R₉C(O)-, R₁₀- (CH₂)ₙ-C(O)-, (R₁₁ₐ)(R_{11b})(R_{11c})C-C(O)O-) ;
(B) un aryle ou Het² (les deux groupes sont facultativement substitués par un à trois groupes choisis parmi alkyle en C₁-C₇, hydroxy, alcoxy en C₁-C₇, halogène, R₁₂ₐOC (O) -, (R_{12b}) (R_{12c}) NC (O) -, R_{12d}C (O) N (R12e) -, R_{12f}C(O)O-, R_{12g}OC (O) NR₁₂ₕ-, (R₁₂ⱼ) (R₁₂ₖ)NC(O)O-, aryle, benzoyle ou Het⁴), R₁₃C (O) -, (R₁₄ₐ)(R_{14b})N-) ;
ou R₁ et R₂ peuvent représenter conjointement une structure cyclique contenant 5 à 14 chaînons, facultativement substituée par un ou plusieurs groupes choisis parmi hydroxy, oxo, alkyle en C₁-C₇ (ledit groupe alkyle est facultativement substitué par un ou plusieurs groupes choisis parmi hydroxy, halogène, aryle ou Het⁷), R₁₅O-, R₁₆ₐOC(O)-, (R_{16b}) (R_{16c}) NC (O) -, R_{16d}C (O) N (R₁₆ₑ) -, R_{16f}C (O) O-, R_{16g}OC (O)NR₁₆ₕ-, (R₁₆ⱼ) (R₁₆ₖ)NC(O)O-, R₁₇C(O)-, aryle ou Het⁵ (lesdits aryle et Het⁵ sont facultativement substitués par l'un ou plusieurs parmi
alkyle en C₁-C₇, hydroxy, oxo alcoxy en C₁-C₇, halogène, R₂₆ₐOC(O)-, (R_{26c}) (R_{26c})NC(O)-, R_{26d}C(O)N(R₂₆ₑ)-, R_{26f}C(O)O-, R_{26g}OC(O)NH-, (R₂₆ₕ) (R₂₆ⱼ)NC(O)O-, phényle ou benzoyle (lesdits phényle ou benzoyle sont facultativement substitués par un ou deux halogènes, (alkyle en C₁-C₅)C(O)O-)), phtalimido, succinimido ou (R₁₈ₐ) (R_{18b}) (R_{18c})C-C(O)O- ;
ou R₁, R₂ et R₂ peuvent représenter conjointement une structure cyclique contenant S à 16 chaînons, facultativement substituée par un ou plusieurs groupes choisis parmi hydroxy, oxo, alkyle en C₁-C₇ (ledit groupe alkyle est facultativement substitué par un ou plusieurs groupes choisi parmi hydroxy, halogène, oxo,
aryle ou Het⁸), R₁₉O-, R₂₀C(O)-, aryle et Het⁶ (lesdits aryle et Het⁶ sont facultativement substitués par un à trois groupes choisis parmi alkyle en C₁-C₇, hydroxy, alcoxy en C₁-C₇, halogène, oxo, R₂₇ₐOC(O)-, (R_{27b}) (R_{27c}) NC (O) -, R_{27d}C (O) N(R₂₇ₑ)-, R_{27f}C(O)O-, R_{27g}OC(O)NH-, (R₂₇ₕ) (R₂₇ⱼ)NC(O)O-, phényle ou benzoyle) , R₂₁ₐOC(O)-, (R_{21b}) (R_{21c})NC(O)-, R_{21d}C (O) N (R21e)-, R_{21f}C(O) O-, R_{21g}OC(O) -NR₂₁ₕ-, (R₂₁ⱼ) (R₂₁ₖ)NC(O)O-, phtalimido, succinimido ou (R₂₂ₐ) (R_{22b}) (R_{22c})C-C(O)O- ;
R₅ est choisi parmi un alkyle en C₁-C₆, un aryle, Het⁹ (lesdits groupes sont facultativement substitués par un ou plusieurs groupes choisis parmi hydroxy, halogène, alcoxy en C₁-C₆) ;
R₆ est choisi parmi un aryle ou Het¹⁰ (les deux groupes étant facultativement substitués par un ou plusieurs groupes choisis parmi alkyle en C₁-C₈, hydroxy, alcoxy en C₁-C₇, halogène, R₂₈ₐOC(O)-, (R_{28b}) (R_{28c}) NC(O)-, R_{28d}C(O)N(R₂₈ₑ) -, R_{28f}C(O)O-, R_{28g}OC (O)-NH-, (R₂₈ₕ) (R₂₈ⱼ)NC(O)O-, aryle, benzoyle ou Het¹¹) ;
R₇ₐ à R₇ₘ sont indépendamment choisis, à chaque occurrence, parmi un hydrogène, un alkyle en C₁-C₇, un aryle ou Het¹² (lesdits alkyle en C₁-C₇, aryle et Het¹² sont facultativement substitués par un ou plusieurs groupes choisis parmi alkyle en C₁-C₆, hydroxy, alcoxy en C₁-C₃, halogène, R₂₉ₐOC(O)-, (R_{23b}) (R_{23c}) NC(O)-, R_{29d}C (O) N (R₂₉ₑ) -, P_{29f}C(O)O-, R_{29g}OC (O)NH-, (R₂₉ₕ) (R₂₉ⱼ)NC(O)O-, aryle, benzoyle ou Het¹³) ;
R₈ est choisi parmi un alkyle en C₁-C₆, un aryle ou Het¹⁴ (lesdits groupes sont facultativement substitués par un ou plusieurs groupes choisis parmi alkyle en C₁-C₆) ;
R₉ est choisi parmi un alkyle en C₁-C₁₂ linéaire ou ramifié (facultativement substitué par R₃₀OC(O)-), un cycloalkyle en C₃-C₁₂ (ledit cycloalkyle est facultativement substitué par un ou plusieurs groupes choisis parmi alkyle en C₁-C₃, hydroxy, alcoxy en C₁-C₃, halogène, R₃₁ₐOC(O)-, (R_{31b}) (R_{31c})NC(O)-, R_{31d}C (O) N (R₃₁ₑ) -, R_{31f}C(O)O-, R_{31g}C(O)N(R₃₁ₕ)-, (R₃₁ⱼ) (R₃₁ₖ)NC(O)O-), aryle, benzoyle ou Het¹⁵), un aryle ou Het¹⁶ (lesdits aryle et Het¹⁶ sont facultativement substitués par un à trois groupes choisis parmi alkyle en C₁-C₆, hydroxy, alcoxy en C₁-C₃, éthylènedioxy, halogène, R₃₂ₐOC(O)-, (R_{32b})(R_{32c})NC(O)-, R_{32d}C (O)NR₃₂ₑ-, R_{32f}C(O)O-, R_{32g}OC(O)NH-, (R₃₂ₕ) (R₃₂ⱼ)NC(O)O-), aryle, benzoyle ou Het¹⁷) ;
R₁₀ est choisi parmi un aryle et Het¹⁸ (lesdits groupes sont facultativement substitués par un à trois groupes choisis parmi alkyle en C₁-C₃, hydroxy, alcoxy en C₁-C₃, halogène, -COOH, éthylènedioxy) ;
R₁₁ₐ est choisi parmi un hydroxy ou -CH₂OH ;
R_{11b} est un phényle (facultativement substitué par un à trois groupes choisis parmi alkyle en C₁-C₃, hydroxy, alcoxy en C₁-C₃, halogène, R₃₃ₐOC(O)-, (R_{33b}) (R_{33c}) NC (O) -, R_{33d}C (O) N (R₃₃ₑ) -, R_{33f}C(O)O-, R_{33g}OC(O)-NH-, (R₃₃ₕ) (R₃₃ⱼ)NC(O)O-) ;
R_{11c} est choisi parmi un hydrogène, un cycloalkyle en C₅-C₆, un phényle (lesdits groupes sont facultativement substitués par un à trois groupes choisis parmi alkyle en C₁-C₃, hydroxy, alcoxy en C₁-C₃, halogène, R₃₄ₐOC (O) -, (R_{34b})(R_{34c})NC(O) -, R_{34d}C(O) N (R₃₄ₑ) -, R_{34f}C(O)O-, R_{34g}OC(O)NH-, (R₃₄ₕ) (R₃₄ⱼ)NC(O)O-) ;
R₁₂ₐ à R₁₂ₖ sont indépendamment choisis, à chaque occurrence, parmi un hydrogène, un alkyle en C₁-C₇, un aryle, Het¹⁹ (lesdits groupes sont facultativement substitués par un ou plusieurs groupes choisis parmi alkyle en C₁-C₆, hydroxy, alcoxy en C₁-C₃, halogène, R₃₅ₐOC(O)-, (R_{35b}) (R_{35c}) NC (O) -, R_{35d}C (O) N (R₃₅ₐ) -, R_{35f}C (0) 0-, R_{35g}OC(O)-NH- , (R_{35b}) (R₃₅ⱼ)NC(O)O-, aryle, benzoyle ou Het²⁰) ;
R₁₃ est choisi parmi un hydrogène ou un alkyle en C₁-C₆ ; R₁₄ₐ à R_{14b} sont indépendamment choisis, à chaque occurrence, parmi un hydrogène ou un alkyle en C₁-C₆ ; R₁₅ est choisi parmi un alkyle en C₁-C₆, un aryle ou Het²¹ (lesdits groupes sont facultativement substitués par un ou plusieurs groupes choisis parmi hydroxy, halogène ou alcoxy en C₁-C₆) ;
R₁₆ₐ à R₁₆ₖ sont indépendamment choisis parmi, à chaque occurrence, un hydrogène, un alkyle en C₁-C₇, un aryle ou Het²² (lesdits groupes sont facultativement substitués par un ou plusieurs groupes choisis parmi alkyle en C₁-C₆, hydroxy, alcoxy en C₁-C₃, halogène, R₃₆ₐOC (O) -, (R_{36b}) (R_{36c}) NC (O) -, R_{36d}C (O) N (R₃₆ₑ) -, R_{36f}C (O) O-, R_{36g}OC(O)-NH-, (R₃₆ₕ) (R₃₆ⱼ) NC (O) O-, aryle, benzoyle ou Het²³) ;
R₁₇ est choisi parmi un hydrogène ou un alkyle en C₁-C₆ ; R₁₈ₐ est choisi parmi un hydroxy ou -CH₂OH ;
R_{18b} est un phényle (facultativement substitué par un à trois groupes choisis parmi alkyle en C₁-C₃, hydroxy, alcoxy en C₁-C₃, halogène, R₃₇ₐOC (O) -, (R_{37b}) (R_{37c}) NC (O) -, R_{37d}C(O)N(R₃₇ₑ)-, R_{37f}C(O)O-, R_{37g}OC(O)NH-, (R₃₇ₕ) (R₃₇ⱼ)NC(O)O-) ;
R_{18c} est choisi parmi un hydrogène, un cycloalkyle en C₅-C₆ ou un phényle (qui est facultativement substitué par un à trois groupes choisis parmi alkyle en C₁-C₃, hydroxy, alcoxy en C₁-C₃, halogène, R₃₈ₐOC (O) -, (R_{38b}) (R_{38c}) NC (O) -, R_{38d}C(O) N (R₃₈ₑ) -, R_{38f}C (O)O-, R_{38g}OC (O) - NH-, (R₃₈ₕ) (R₃₈ⱼ)NC(O)O-) ;
R₁₉ est choisi parmi un alkyle en C₁-C₆, un aryle ou Het²⁴ (lesdits groupes sont facultativement substitués par un ou plusieurs groupes choisis parmi hydroxy, halogène ou alcoxy en C₁-C₆) ;
R²⁰ est choisi parmi un hydrogène et un alkyle en C₁-C₆ ; R₂₁ₐ à R₂₁ₖ sont indépendamment choisis, à chaque occurrence, parmi un hydrogène, un alkyle en C₁-C₇, un aryle ou Het²⁵ (lesdits groupes sont facultativement substitués par un ou plusieurs groupes choisis parmi alkyle en C₁-C₆, hydroxy, alcoxy en C₁-C₃, halogène, R₃₉ₐOC(O)-, (R_{39b})(R_{39c})NC(O)-, R_{39d}C (O) N (R₃₉ₑ)-, R_{39f}C(O)O-, R_{39g}OC (O) -NH-, (R₃₉ₕ) (R₃₉ⱼ) NC (O) O-, aryle, benzoyle ou Het²⁶) ;
R₂₂ₐ est choisi parmi un hydroxy ou -CH₂OH ;
R_{22b} est un phényle (facultativement substitue par un à trois groupes choisis parmi alkyle en C₁-C₃, hydroxy, alcoxy en C₁-C₃, halogène, R₄₀ₐOC (O) -, (R_{40b}) (R_{40c}) NC(O) -, R_{40d}C(O) N (R₄₀ₑ) -, R_{40f}C (O) O-, R_{40g}OC (O) NH-, (R₄₀ₕ) (R₄₀ⱼ)NC(O)O-) ;
R_{22c} est choisi parmi un hydrogène, un cycloalkyle en C₅-C₆ ou un phényle (qui est facultativement substitué par un à trois groupes choisis parmi alkyle en C₁-C₃,
hydroxy, alcoxy en C₁-C₃, halogène, R₄₁ₐOC (O) -, (R_{41b}) (R_{41c}) NC (O) -, R_{41d}C (O)N (R₄₁ₑ) -, R_{41f}C (O) O-, R_{41g}OC (O) NH-, (R₄₁ₕ) (R₄₁ⱼ) NC (O) O-) ;
R₂₃ₐ à R₂₃ⱼ sont indépendamment choisis, à chaque occurrence, parmi un hydrogène ou un alkyle en C₁-C₆ ;
R₂₄ₐ à R₂₄ⱼ sont indépendamment choisis, à chaque occurrence, parmi un hydrogène ou un alkyle en C₁-C₆;
R₂₅ est choisi parmi un alkyle en C₁-C₄, un aryle ou Het²⁷ (lesdits aryle et Het²⁷ sont facultativement substitués par un ou deux halogènes, alkyle en C₁-C₄, hydroxy(alkyle en C₁-C₄), alcoxy en C₁-C₄, hydroxy(alcoxy en C₁-C₄), nitro) ;
R₂₆ₐ à R₂₆ⱼ sont indépendamment choisis, à chaque occurrence, parmi un hydrogène ou un alkyle en C₁-C₆ ;
R₂₇ₐ à R₂₇ⱼ sont indépendamment choisis, à chaque occurrence, parmi un hydrogène ou un alkyle en C₁-C₆ ;
R₂₈ₐ à R₂₈ⱼ sont indépendamment choisis, à chaque occurrence, parmi un hydrogène ou un alkyle en C₁-C₆ ;
R₂₉ₐ à R₂₉ⱼ sont indépendamment choisis, à chaque occurrence, parmi un hydrogène ou un alkyle en C₁-C₆ ;
R₃₀ est choisi parmi un hydrogène ou un alkyle en C₁-C₆ ;
R₃₁ₐ à R₃₁ₖ sont indépendamment choisis, à chaque occurrence, parmi un hydrogène ou un alkyle en C₁-C₆ ;
R₃₂ₐ à R₂₂ⱼ sont indépendamment choisis, à chaque occurrence, parmi un hydrogène ou un alkyle en C₁-C₆ ;
R₃₃ₐ à R₃₃ⱼ sont indépendamment choisis, à chaque occurrence, parmi un hydrogène ou un alkyle en C₁-C₆ ;
R₃₄ₐ à R₃₄ⱼ sont indépendamment choisis, à chaque occurrence, parmi un hydrogène ou un alkyle en C₁-C₆ ;
R₃₅ₐ à R₃₅ⱼ sont indépendamment choisis, à chaque occurrence, parmi un hydrogène ou un alkyle en C₁-C₆ ;
R₃₆ₐ à R₃₆ⱼ sont indépendamment choisis, à chaque occurrence, parmi un hydrogène ou un alkyle en C₁-C₆ ;
R₃₇ₐ à R₃₇ⱼ sont indépendamment choisis, à chaque occurrence, parmi un hydrogène ou un alkyle en C₁-C₆ ;
R₃₈ₐ à R₃₈ⱼ sont indépendamment choisis, à chaque occurrence, parmi un hydrogène ou un alkyle en C₁-C₆ ;
R₃₉ₐ à R₃₉ⱼ sont indépendamment choisis, à chaque occurrence, parmi un hydrogène ou un alkyle en C₁-C₆ ;
R₄₀ₐ à R₄₀ⱼ sont indépendamment choisis, à chaque occurrence, parmi un hydrogène ou un alkyle en C₁-C₆ ;
R₄₁ₐ à R₄₁ⱼ sont indépendamment choisis, à chaque occurrence, parmi un hydrogène ou un alkyle en C₁-C₆ ; Les groupes (Het¹ à Het²⁷) comprennent les systèmes cycliques ayant un nombre total d'atomes dans le système cyclique compris entre cinq et douze atomes et contenant de 1 à 5 hétéroatomes (choisis parmi N, 0 et S) ;
m est un entier choisi parmi 1 à 5 ;
n est un entier choisi parmi 1 à 3 ;
ledit procédé comprend les étapes suivantes :
(i) : mélange d'ésoméprazole et de N⁺(R₁) (R₂) (R₃) (R₄) X⁻ ;
où
R₁, R₂, R₃ et R₄ sont tels que définis ci-dessus ;
X⁻ est choisi parmi Cl⁻' Br⁻' I⁻' des carboxylates d'alkyle en C₁-C₆, des sulfonates d'alkyle en C₁-C₆, HSO₄⁻ et OH⁻ ;
dans un système de solvant aqueux comprenant plus de 40 % m/m de carbonate de potassium ;
(ii) : ajout d'un solvant hydrocarbure chloré immiscible dans l'eau ;
(iii) : isolement de la phase organique ;
(iv) : récupération du composé de formule I.

2. Procédé selon la revendication 1 **caractérisé en ce que** le système de solvant aqueux dans l'étape (i) comprend plus de 50 % en poids de carbonate de potassium.

3. Procédé selon la revendication 1 **caractérise en ce que** le système de solvant aqueux dans l'étape (i) est saturé avec du carbonate de potassium.

4. Procécé selon la revendication 1 **caractérisé en ce que** le solvant chloré immiscible dans l'eau est choisi parmi le 1,2-dichlorométhane, le trichlorométhane et le 1,2-dichloroéthane.

5. Procédé selon la revendication 1 **caractérisé en ce que** l'ésoméprazole dans l'étape (i) est le sel de sodium ou le sel de potassium d'ésoméprazole.

6. Procédé selon l'une quelconque des revendications 1 et 5 **caractérisé en ce que** R₂, R₃ et R₄ sont individuellement choisis parmi, à chaque occurrence, un groupe alkyle en C₁-C₆ linéaire ou ramifié.

7. Procédé selon la revendication 1 **caractérisé en ce que** R₁ est choisi parmi un alkyle en C₁-C₈ linéaire ou ramifié (ledit groupe alkyle est facultativement substitué par un ou plusieurs groupes choisis parmi amino, hydroxy, halogène, R₅O- ou aryle) ; R₂, R₃ et R₄ sont individuellement choisie parmi un groupe alkyle en C₁-C₄ linéaire ou ramifié (ledit groupe alkyle est facultativement substitué par un ou plusieurs groupes choisis parmi amino, hydroxy, halogène ou R₅O-) ou un aryle.

8. Procédé selon la revendication 1 **caractérisé en ce que** R₁ est choisi parmi un groupe alkyle en C₁-C₈ linéaire ou ramifié, ledit groupe alkyle est facultativement substitué par un ou plusieurs groupes choisis parmi amino, hydroxy, halogène, R₅O- ou phényle ; R₂, R₃ et R₄ sont indépendamment, à chaque occurrence, choisis parmi un méthyle, un éthyle, un n-propyle ou un isopropyle (ledit groupe est facultativement substitué par un ou plusieurs groupes choisis parmi amino, hydroxy, halogène ou R₅O-) ou un phényle.

9. Procédé selon la revendication 1 **caractérisé en ce que** R₁ et R₂ peuvent représenter conjointement une structure cyclique contenant 5 à 10 chaînons, facultativement substituée par un ou plusieurs groupes choisis parmi alkyle en C₁-C₅ linéaire ou ramifié, amino, hydroxy, halogène ou R₅O- ; R₃ et R₄ sont choisis parmi un groupe alkyle en C₁-C₄ linéaire ou ramifié.
